# EUROPEAN PATENT APPLICATION

(11) **EP 4 420 659 A1**
(43) Date of publication of application: **28.08.2024**
(21) Application number: 23305242.2
(22) Date of filing: 23.02.2023
(51) Int. Cl.: A61K 31/223, A61K 31/336, A61P 9/00, A61P 9/04

(54) **SPECIFIC VASH COMPOUNDS (SVC) INHIBITORS FOR HEART DISEASES**

(71) Applicant: Mt Act, 34960 Montpellier Cedex 02 (FR); The Trustees of the University of Pennsylvania, Philadelphia, PA 19104-4310 (US)
(72) Inventor: VAN DER LAAN, Siem, 34190 CAZILHAC (FR); HACHED, Khaled, 34090 MONTPELLIER (FR); PROSSER, Benjamin L., SWARTHMORE, 19081 (US); MARGULIES, Kenneth B., VILLANOVA, 19085 (US)
(74) Representative: Regimbeau

(57) **Abstract**

The present invention relates to a compound of formula (I) : 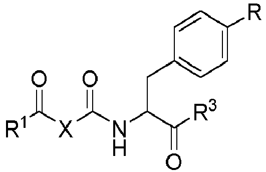 or a pharmaceutically acceptable salt and/or solvate thereof,
for use in the prevention and/or the treatment of heart failure, cardiomyopathies, myocardial infarction induced cardiac dysfunction and/or for improving heart function in a subject in need thereof.

## Description

### Field of the invention

The present invention relates to new Specific Vash Compounds (SVC) in the prevention and/or the treatment of heart diseases, in particular heart failure and cardiomyopathies.

### Background of the invention

Heart Failure (HF) is defined as a failure of the heart to provide adequate blood flow to the organs and tissues of the body. Heart failure is often characterized by diastolic dysfunction, or inadequate ventricular filling due to the inability of the myocardium to fully relax. Diastolic dysfunction is a hallmark of hypertrophic cardiomyopathy (HCM) (Maron et al., 1995) and heart failure with preserved ejection fraction (HFpEF). These conditions often are characterized by prolonged and impaired left ventricular (LV) relaxation, slow LV filling, and increased diastolic LV stiffness. The prevalence of Heart Failure continues to rise, and its significant mortality underscores the need for new therapies. In particular, Heart failure with preserved ejection fraction (HFpEF), currently has limited therapeutic options, even though it is estimated to represent about half of all heart failure cases. Many cases of HFpEF exhibit slowed relaxation of the heart muscle that contributes to abnormalities of pump function. Slowed myocardial relaxation in HFpEF is also due to increased internal resistance attributable to densification of microtubules (MTs) and stabilising posttranslational modifications such as alpha tubulin detyrosination (Chen et al., 2018). Hence targeting MTs to reduce overall cardiomyocyte stiffness might improve relaxation rates without increasing energy usage.

Microtubules (MTs) are a major cytoskeleton element found in all eukaryotic cells. They are formed by polymerization of alpha- and beta-tubulin heterodimers and function as mechanotransducers converting changing contractile forces into intracellular signals. Also, MTs act as compression resistant elements, which provide a mechanical impediment to cardiomyocyte contraction. They act as viscoelastic resistance elements that can impair sarcomere shortening and thus cardiac function, particularly in disease states associated with MT proliferation. Posttranslational modification (PTM) of MTs modifies their mechanical properties and binding interactions (Roll-Mecak, 2020). Detyrosination, a PTM of alpha-tubulin, has recently been shown to impact MT-dependent mechanotransduction in both cardiac and skeletal muscle diseases (Kerr J.P et al., 2015). This specific PTM is markedly increased in patient tissue and preclinical models of heart disease (Chen et al., 2018). In failing myocardium, the maladaptation of a cardiomyocyte to chronic stress correlates with densification of the MT network and increased VASH protein abundance and/or detyrosinating activity. Densification of the MT network and disease-related exacerbation of the detyrosinating activity mechanically interfere with cardiomyocytes function by impairing contraction, thus leading to compensatory mechanisms and profound remodelling of the myocardium.

There remains a high pressing need for developing safe and effective treatments of heart failure, and particularly treatments that minimize the increased risk of ischemia or arrhythmias associated with current palliative efforts which come at high price. Over the last five years, researchers identified modified cardiomyocyte (CM) microtubules (MTs) as a novel therapeutic target for the treatment of HF. In human and murine HF of diverse origin, there is a marked densification of microtubules and increase of detyrosinating activity (Tsutsui H. et *al,* 1993; Cheng G. et al., 2008) which leads to cardiomyocyte stiffness and impaired contractile motion. Reversing this posttranslational modification by forcing expression of the reverse enzyme called Tubuline Tyrosine Ligase (TTL) is sufficient to lower stiffness and improves contractility in failing human and murine cardiomyocytes and myocardium. However, the use of viral expression vectors for cardiovascular application remains complicated. Of note, and of interest for the cardiovascular medical knowledge, addressing detyrosination status by TTL-expression leads to improvements in cardiac mechanics that occur independently of calcium cycling, strongly de-risking the new therapeutic approach of reducing detyrosination.

It was shown that gene therapy to reduce detyrosination can improve cardiac function in the heart failure setting. While cardiac gene therapy holds significant potential, it faces many hurdles, particularly in large animals, where achieving safe and efficient transduction of the heart is a major challenge.

In parallel, inhibition of the detyrosinating enzyme demonstrates considerable therapeutic potential. The vasohibin enzymes (VASH1 and 2), in concert or not with their chaperone small vasohibin binding protein (SVBP), remove the C-terminal tyrosine residue from alpha tubulin of polymerized MTs (Van der Laan S. et al., 2019). VASH1 was recently identified as the primary detyrosinase in human and murine myocardium, and it was found that VASH1 KD is sufficient to lower CM stiffness, improve fractional shortening, and speed contraction and relaxation in failing human CMs. Of particular note, VASH1 KD led to a markedly faster relaxation in CM from patients with Heart Failure with preserved Ejection Fraction (HFpEF), independent of alterations in the Ca²⁺ transient (Chen, C. Y. *et al.,* 2020). Because VASH1 does not bind and sequester free tubulin, VASH1 KD demonstrates no overt MT depolymerizing effect in CMs (Chen, C. Y. *et al.,* 2020), and instead specifically reduces dTyr while preserving network dynamics and density. Preventing VASH2-dependent detyrosination substantially limits the reduction in the left ventricular ejection fraction after acute myocardial infarction in mice, without affecting infarct size or cardiac remodeling (Yu X. et al., 2021).

Currently available VASH inhibitors are flawed but improving. Parthenolide (PTL), a highly reactive sesquiterpene lactone that is an FDA-approved chemotherapeutic agent, indeed inhibit VASH activity directly (Aillaud, C. *et al.,* 2017), albeit only at high concentrations. In CMs, 10microM PTL (below its IC50) produces a modest (30-40%) reduction in dTyr after 2 hours of treatment (Kerr, J. P. *et al.* , 2015; Robison, P. *et al.* , 2016; Schuldt, M. & Kuster, 2020), but has numerous potential off-target effects that limit its prolonged use, including modulation of NF-kB, STAT3, and JNK signaling pathways (Caporizzo, M. A., Chen, C. Y. & Prosser, B. L, 2019) and is not transferrable to a product development.

So, there is still a need to provide more Specific VASH Compounds (SVC) inhibitors.

In 2017, epoxide-Y (Epo-Y) was described and used in a targeted chemical proteomics screen to both identify the detyrosinating enzyme complex and to function as a >10-fold more potent VASH inhibitor than PTL (Aillaud, C. *et al.,* 2017).

But the inventors recently developed SVC inhibitors showing a 10-1000-fold improvement in VASH inhibition compared to Epo-Y, and readily cross cell membranes to effectively reduce dTyr in cells and improve cardiomyocytes function. The inventors tested these compounds on the ZSF1 Obese Rat, which is considered to be the best current rodent model of Heart Failure with preserved Ejection Fraction (HFpEF) as it presents 3 key co-morbidities associated with human HFpEF, which are hypertension, obesity, and diabetes. And they demonstrated that these compounds effectively improve diastolic function. In particular, they demonstrated that the SVC inhibitors significantly lowered myocyte stiffness and improved the kinetics of contraction and relaxation in ZSF1-Obese myocytes relative to ZSF1-Lean controls. And advantageously, these compounds are overtly tolerated and effective at reducing detyrosination when delivered by intravenous or *per* os administration *in vivo.*

### Summary of the present invention

So the present invention concerns compositions and methods useful for treating patients with heart failure and/or cardiomyopathies, in particular patients with heart failure with reduced or preserved ejection fraction (e.g., HFrEF or HFpEF, respectively) and/or useful for lowering cardiac stiffness and for improving contractility, in a Ca²⁺ independent manner. The compositions and methods provided herein may be combined with other therapies ('combination therapy'), including those which involve Ca²⁺- dependent pathways.

A first aspect of the invention relates to a method for improving heart function in a subject in need thereof, comprising administering to a subject in need thereof of a compound of formula (I) 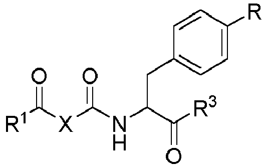
or a pharmaceutically acceptable salt and/or solvate thereof,
in which
X is 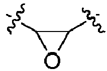 or -NH-CH₂-
R1 is 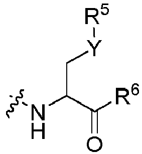 or NR^{1a}R^{1b} with R^{1a} is H and R^{1b} is C₁- C₆ alkyl non substituted or substituted with C(O)-O-C₁-C₆ alkyl or C(O)-NH-C₁-C₆ alkyl,
R is O-R² with R² is a C₁-C₆ aliphatic chain, said aliphatic chain being optionally substituted, or C₁-C₆ alkyl-aryl,
R³ is OH or O-C₁-C₆ aliphatic chain,
Y is -(CH₂)ₘ- with m=2 or 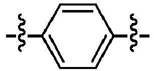
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain.

In other words, the present invention also relates to a compound of formula (I) : 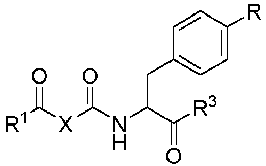
or a pharmaceutically acceptable salt and/or solvate thereof,
in which
X is 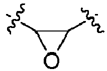 or -NH-CH₂-
R1 is 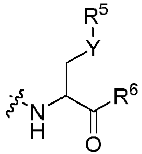 or NR^{1a}R^{1b} with R^{1a} is H and R^{1b} is C₁- C₆ alkyl non substituted or substituted with C(O)-O-C₁-C₆ alkyl or C(O)-NH-C₁-C₆ alkyl,
R is O-R² with R² is a C₁-C₆ aliphatic chain, said aliphatic chain being optionally substituted, or C₁-C₆ alkyl-aryl,
R³ is OH or O-C₁-C₆ aliphatic chain,
Y is -(CH₂)ₘ- with m=2 or 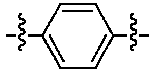
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain,
for use in the prevention and/or the treatment of heart failure, cardiomyopathies myocardial infarction induced cardiac dysfunction and/or for improving heart function in a subject in need thereof.

Another aspect of the invention is a method treating cardiovascular disease selected from heart failure, cardiomyopathies, and myocardial infarction induced cardiac dysfunction, in a subject in need thereof, administering to a subject in need thereof of an effective dose of a compound of formula (I) 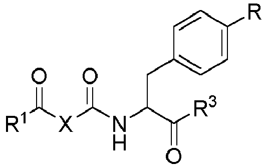
or a pharmaceutically acceptable salt and/or solvate thereof,
in which
X is 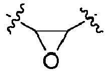 or -NH-CH₂-
R1 is 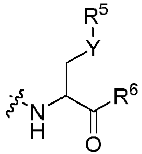 or NR^{1a}R^{1b} with R^{1a} is H and R^{1b} is C₁- C₆ alkyl non substituted or substituted with C(O)-O-C₁-C₆ alkyl or C(O)-NH-C₁-C₆ alkyl,
R is O-R² with R² is a C₁-C₆ aliphatic chain, said aliphatic chain being optionally substituted, or C₁-C₆ alkyl-aryl,
R³ is OH or O-C₁-C₆ aliphatic chain,
Y is -(CH₂)ₘ- with m=2 or 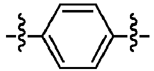
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain.

Another aspect of the invention is a combination therapy wherein the compound (I) as defined in the invention is combined with a current standard-of-care treatment for heart failure, cardiomyopathies, and/or myocardial infarction induced cardiac dysfunction.

A still another aspect of the invention is a use of the compound (I) as defined above or a conjugate comprising a fragment of a compound of formula (I) linked or not to a biomolecule optionally labeled, as research tool for research and development activities in heart function disorders or heart failure.

### Detailed description

### Definitions

Unless defined otherwise in this specification, technical and scientific terms used herein have the same meaning as commonly understood by one of ordinary skill in the art.

The term **"a"** or **"an"** refers to one or more. As such, the terms "a" (or "an"), "one or more," and "at least one" are used interchangeably in the present description.

The words **"comprise", "comprises",** and **"comprising"** are to be interpreted inclusively rather than exclusively. The words **"consist", "consisting",** and its variants, are to be interpreted exclusively, rather than inclusively. While various embodiments in the specification are presented using "comprising" language, under other circumstances, a related embodiment is also intended to be interpreted and described using "consisting of or "consisting essentially of language.

The term **"about"** encompasses a variation within and including + 10% unless otherwise specified.

As used herein, the term **"therapy"** refers to any protocol, method and/or agent that can be used in the prevention, management, treatment and/or amelioration of a disease, disorder, or condition in a patient. The patient may be at risk of contracting the disease, disorder, or condition, or suspected to have contracted the disease, disorder, or condition. Alternatively, the patient may have been diagnosed as suffering from the disease, disorder or condition. Non-limiting examples of therapy comprise administration of a composition (e.g., a pharmaceutical composition), physical therapy, physiotherapy, psychological therapy, etc. Therapy also includes the possibility to combine several therapies. In this case, the various therapies may be sequential, simultaneous, or intermixed. When therapy comprises administering or the administration of a composition, the composition is administered in an amount, manner, and/or mode effective to treat or prevent the patient's disease, disorder, or condition. Therapy may require administration of the composition more than once.

As used herein, the term **"treating"** or **"treatment"** means an improvement of the patient's disease, disorder, or condition, which may be observed at the clinical, histological, and/or biochemical level. The terms "treating" or "treatment" notably include improving a clinical, histological, and/or biochemical symptom or parameter associated with the patient's disease, disorder, or condition or inhibiting, reducing, or delaying progression or exacerbation of the patient's disease, disorder, or condition (including secondary damage caused by the disease, disorder, or condition) to either a statistically significant degree or to a degree detectable to one skilled in the art. In some embodiments, treatment is assessed on a population basis such that a therapy is considered to "treat" a particular disease, disorder or condition if a statistically significant improvement of the patient's disease, disorder, or condition is observed in a population suffering from the disease, disorder, or condition.

As used herein, the terms **"prevent", "prevention" and "preventing"** refer to the reduction in the risk for a subject of acquiring or developing a given disease, disorder, or condition. The terms "prevent", "prevention" and "preventing" also comprise delaying the onset and/or reducing the frequency and/or intensity of clinical, histological and/or biochemical symptoms or parameters associated with this given disease, disorder, or condition. In some embodiments, prevention is assessed on a population basis such that a therapy is considered to "prevent" a particular disease, disorder or condition if a statistically significant decrease in the risk of acquiring or developing the disease, disorder, or condition, and/or a statistically significant delay of the onset and/or a statistically significant decrease of the frequency and/or intensity of clinical, histological and/or biochemical symptoms or parameters associated with the disease, disorder or condition, is observed in a population susceptible to the disease, disorder, or condition.

As used herein, the term **"administering"** means oral administration, administration as a suppository, topical contact, intravenous, parenteral, intraperitoneal, intramuscular, intralesional, intrathecal, intracranial, intranasal or subcutaneous administration, or the implantation of a slow-release device, e.g., a mini-osmotic pump, to a subject. Administration is by any route, including parenteral and transmucosal (e.g., buccal, sub lingual, palatal, gingival, nasal, vaginal, rectal, or transdermal). Parenteral administration includes, e.g., intravenous, intramuscular, intra-arterial, intradermal, subcutaneous, intraperitoneal, intraventricular, and intracranial. Other modes of delivery include, but are not limited to, the use of liposomal formulations, intravenous infusion, transdermal patches, etc.

By **"co-administer"** it is meant that a composition described herein is administered at the same time, just prior to, or just after the administration of one or more additional therapies. In certain embodiments, the composition is specifically targeted (e.g., via direct injection) to the heart. The term **"combination"** as used herein refers to any arrangement possible of various components (e.g. the compound of formula (I) according to the invention and another treatment). Such an arrangement includes mixture of said components as well as separate combinations for concomitant or sequential administrations. The present invention encompasses combinations comprising equal molar concentrations of each component as well as combinations with very different concentrations. It is appreciated that optimal concentration of each component of the combination can be determined by the artisan skilled in the art.

By **'current standard-of-care treatment** for heart failure, cardiomyopathies, and myocardial infarction induced cardiac dysfunction', it means accepted treatment regimens for cardiovascular disease selected from heart failure, cardiomyopathies, and myocardial infarction induced cardiac dysfunction, such as the ones listed in the 2021 ESC Guidelines for the diagnosis and treatment of acute and chronic heart failure (McDonagh T.A et al., 2021).

By **'a subject in need thereof',** it means animals, preferably mammals including humans. In a particular and preferred embodiment, the subject in need thereof is humans having or susceptible to develop heart disorders, in particular heart failure, cardiomyopathies or and myocardial infarction induced cardiac dysfunction.

**'Heart failure'** is a condition that develops when the heart doesn't pump enough blood for body's needs. This can happen if the heart can't fill up with enough blood. It can also happen when the heart is too weak to pump properly.

Heart failure can develop suddenly (the acute kind) or over time as your heart gets weaker (the chronic kind). It can affect one or both sides of the heart. Left-sided and right-sided heart failure may have different causes. Most often, heart failure is caused by another medical condition that damages your heart, including coronary heart disease and subsequent myocardial infarction, heart-inflammation, high blood pressure, cardiomyopathy, or an irregular heartbeat. Heart failure can damage liver or kidneys. Other conditions it can lead to include pulmonary hypertension or other heart conditions, such as an irregular heartbeat, heart valve disease, and sudden cardiac arrest.

**'Cardiomyopathy'** refers to problems with the heart muscle that can make it harder for the heart to pump blood. There are many types and causes of cardiomyopathy, and it can affect people of all ages. Depending on the type of cardiomyopathy, the heart muscle may become thicker, stiffer, or larger than normal. This can weaken the heart and cause an irregular heartbeat, heart failure, or even cardiac arrest. It encompasses dilated, hypertrophic, ischemic, genetic, and idiopathic cardiomyopathies.

In a particular embodiment, the heart failure or cardiomyopathies in the present invention encompasses **chronic heart failure or cardiomyopathies.**

In another particular embodiment, the heart failure or cardiomyopathies in the present invention encompasses **acute heart failure** (AHF) syndromes including post-myocardial infarction, post-cardiotomy states, following cardiac arrest, in the setting of hypertensive crises, acute presentations of non-ischemic cardiomyopathies, and acute exacerbations of chronic cardiomyopathies of various etiologies.

The term **"stereoisomers"** used in this invention refers to configurational stereoisomers and more particularly to optical isomers.

In the present invention, the optical isomers result in particular from the different position in space of the substituents attached to X. The carbon atoms or the nitrogen atoms of the X group to which the substituents are attached thus represent chiral or asymmetric centres. Optical isomers that are not mirror images of one another are thus designated as "diastereoisomers", and optical isomers, which are non-superimposable mirror images are designated as "enantiomers".

An equimolar mixture of two enantiomers of a chiral compound is designated as a racemic mixture or racemate.

In the context of the present invention, depending of the position of the substituents attached to the X group, the compound of the present invention may be a diastereoisomer of configuration (S,S), of configuration (R,R), of configuration (S,R) or of configuration (R,S) as illustrated hereafter

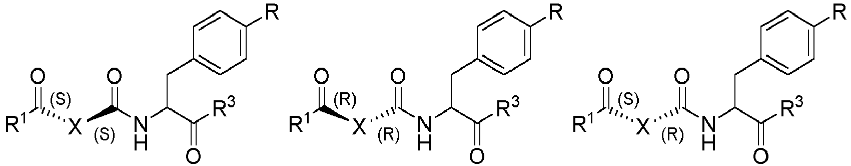

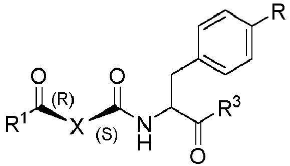

When the position of the substituents is not specified in the compound, said compound corresponds to any one of the above diastereoisomers or to a mixture of said diastereoisomers. In a particular embodiment, the compounds used in the present invention are diastereoisomer of configuration (S,S).

The term **"pharmaceutical composition"** is meant in the framework of the present invention a composition having preventive and curative properties.

For the purpose of the invention, the term **"pharmaceutically acceptable"** is intended to mean what is useful to the preparation of a pharmaceutical composition, and what is generally safe and non-toxic, for a pharmaceutical use.

The term **"pharmaceutically acceptable salt and/or solvate"** is intended to mean, in the framework of the present invention, a salt and/or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L25 tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

The term **"Cₓ-C_{y} aliphatic chain"** designates a linear or branched hydrocarbon chain, completely saturated or containing one or more unsaturations, but not aromatic, comprising from x to y carbon atoms, notably from 1 to 12 carbon atoms, preferably from 1 to 6 carbon atoms. According to the present invention, the term "aliphatic chain" includes substituted or unsubstituted, linear or branched, alkyl, alkenyl or alkynyl groups.

The term **"C₁-C₆ alkyl",** as used in the present invention, refers to a straight or branched monovalent saturated hydrocarbon chain containing from 1 to 6 carbon atoms including, but not limited to, methyl, ethyl, n-propyl, iso-propyl, n-butyl, iso-butyl, sec-butyl, t-butyl (also named ter-butyl), n-pentyl, n-hexyl, and the like.

The term **"aryl",** as used in the present invention, refers to an aromatic hydrocarbon group preferably comprising from 6 to 12 carbon atoms and comprising one or more fused rings, such as, for example but not limited to, a phenyl or naphthyl group. Advantageously, it is a phenyl group.

The term **"C₁-C₆-alkyl aryl"** as used in the present invention, refers to an alkyl group as defined above substituted respectively by an aryl as defined above. Advantageously, a "C₁-C₆-alkyl aryl" is a benzyl group.

In the context of the present invention, **"optionally substituted"** means that the group in question is optionally substituted with one or more substituents which may be selected in particular from C₁-C₆ alkyl, NR^{a}R^{b}, COR^{c}, CO₂R^{d}, CONR^{e}R^{f}, OR^{g}, N⁺R^{h}RⁱR^{j}, wherein R^{a} to R^{j} are, independently of one another, H, C₁-C₆ alkyl or aryl, preferably H or C₁-C₆ alkyl.

The term **"peptide coupling"** refers to a chemical reaction between an amine function and a carboxylic acid function. The peptide coupling will be advantageously carried out in the presence of a coupling agent, such as diisopropylcarbodiimide (DIC), dicyclohexylcarbodiimide (DCC), 1-(3-dimethylaminopropyl)-3-ethylcarbodiimide hydrochloride (EDC), carbonyldiimidazole (CDI), hexafluorophosphate 2-(1H-benzotriazole-1-yl)-1,1,3,3-tetraméthyluronium (HBTU), tetrafluoroborate 2-(1H-benzotriazole-1-yl)-1,1,3,3-tetramethyluronium (TBTU), hexafluorophosphate O-(7-azobenzotriazol-1-yl)-1,1,3,3-tetramethyluronium (HATU), (benzotriazol-1-yloxy)tripyrrolodinophosphonium hexafluorophosphate (PyBOP), 7-Azabenzotriazol-1-yloxy)tripyrrolidinophosphonium hexafluorophosphate (PyAOP) or propylphosphonic anhydride; optionally associated with an additive or a base, such as N-hydroxysuccinimide (NHS), N-hydroxy-benzotriazole (HOBt), 3,4-dihydro-3-hydroxy-4-oxo-1,2,3-benzotriazole (HOOBt), I-hydroxy-7-azabenzotriazole (HAt), N-hydroxysylfosuccinimide (sulfo NHS), dimethylaminopyridine (DMAP), diisopropylethylamine (DIEA) or N-methylmorpholine (NMM).

The term **"VASH or Vasohibin (enzyme)",** as used in the present invention, refers to a tubulin carboxypeptidase enzyme (TCPase) involved in microtubules detyrosination mechanisms associated with muscular dystrophies and in particular heart failure, cardiomyopathies and myocardial infarction induced cardiac dysfunction.

The term **"biomolecule"** refers to a molecule having biological properties. In the context of the present invention, it refers to a protein, a peptide, a biomarker, such as but not limited to photolabeling agents.

The term **"prodrug"** relates to a typically pharmacologically inactive or less active derivative of an active drug that undergoes *in cellulo* or *in vivo* biotransformation to release the active drug by chemical or enzymatic cleavages. By "pharmacologically inactive or less active derivative", it is understood, in the context of the invention, that the prodrug does not have relevant activity with respect to the inhibition of the VASH active site in an *in vitro* setting. However, they are active in *in cellulo* and *in vivo* assays because such tests allow the transformations required to provide the active drug. Prodrugs can offer many advantages over parent drugs such as increased cellular penetration, solubility, enhanced stability, improved bioavailability, reduced side effects, and better selectivity. Activation of prodrugs can involve many enzymes including, but not limited to, oxidoreductases like CYP450 and DT-diaphorase as well as hydrolytic enzymes like carboxylesterase and β-glucuronidase.

In the context of the invention, the prodrug compounds as defined in the present disclosure may be inactive *in vitro.* However, the prodrug compounds possess increased cell penetration. After cell penetration, the prodrug compounds are hydrolyzed to provide the corresponding active drugs, i.e. the potent VASH inhibitors.

So, a first aspect of the invention is a method for improving heart function in a subject in need thereof, comprising administering to a subject in need thereof of a compound of formula (I) 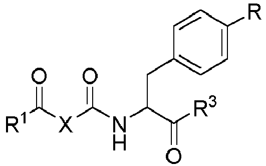
or a pharmaceutically acceptable salt and/or solvate thereof,
in which
X is 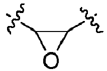 or -NH-CH₂-
R1 is 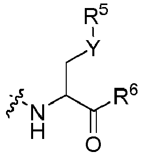 or NR^{1a}R^{1b} with R^{1a} is H and R^{1b} is C₁- C₆ alkyl non substituted or substituted with C(O)-O-C₁-C₆ alkyl or C(O)-NH-C₁-C₆ alkyl ,
R is O-R² with R² is a C₁-C₆ aliphatic chain, said aliphatic chain being optionally substituted, or C₁-C₆ alkyl-aryl,
R³ is OH or O-C₁-C₆ aliphatic chain,
Y is -(CH₂)ₘ- with m=2 or 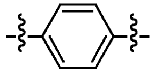
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain.

In other words, the present invention relates to a compound of formula (I) : 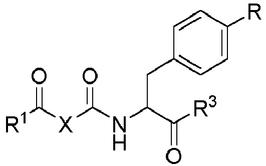
or a pharmaceutically acceptable salt and/or solvate thereof,
in which
X is 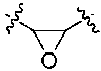 or -NH-CH₂-
R1 is 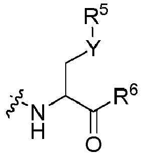 or NR^{1a}R^{1b} with R^{1a} is H and R^{1b} is C₁- C₆ alkyl non substituted or substituted with C(O)-O-C₁-C₆ alkyl , or C(O)-NH-C₁-C₆ alkyl ,
R is O-R² with R² is a C₁-C₆ aliphatic chain, said aliphatic chain being optionally substituted, or C₁-C₆ alkyl-aryl,
R³ is OH or O-C₁-C₆ aliphatic chain,
Y is -(CH₂)ₘ- with m=2 or 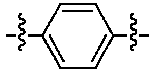
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain, for use in the prevention and/or the treatment of heart failure, cardiomyopathies, myocardial infarction induced cardiac dysfunction, and/or for improving heart function in a subject in need thereof.

### Compound of formula (I) used in the present invention

The compound according to the present invention can be in the form of a stereoisomer or a mixture of stereoisomers, such as a mixture of enantiomers or diastereoisomers, notably a racemic mixture. According to a specific embodiment, the compound of formula (I) is in the form of one diastereoisomer of configuration (S,S), of configuration (R,R), of configuration (S,R) or of configuration (R,S) as defined above.

The formula (I) as defined herein encompasses both active compounds, i.e drugs, and prodrugs thereof.

In particular, the term "prodrug" according to the present invention refers to compounds of formula (I) in which R³ is not OH.

Preferably, the prodrugs according to the present invention are compounds of formula (I) in which R³ is O-C₁-C₆ aliphatic chain, such as O-C₁-C₆ alkyl, in particular O-ethyl, O-isopropyl or O-terbutyl.

In the context of the present invention, the term "drug" refers to compounds of formula (I) in which R³ is OH.

Typically, R³ together with the adjacent carboxyl group forms an ester group. When being converted from the prodrug to the drug, this ester formed by R³ together with the adjacent carboxyl group is typically hydrolyzed in the corresponding carboxylic acid.

In the context of the present invention, the drug differs from its corresponding prodrug in that the ester groups presents in the prodrug are converted to corresponding carboxylic acids in the drug. Preferably, the prodrug comprises only one ester group being formed by R³ together with the adjacent carboxyl group. Therefore, the drug corresponding to a given prodrug has typically the same formula as said prodrug except the R³ group. In other words, in a given prodrug and the drug thereof, substituents R¹, X and R are typically respectively identical. Alternatively, the prodrug may comprise two or more ester groups. In such case, all the ester groups are converted to carboxylic acids in the corresponding drug.

Preferably, the compound of formula (I) is in the form of a diastereoisomer of configuration (S,S) and responds to the following formula (I-A) : 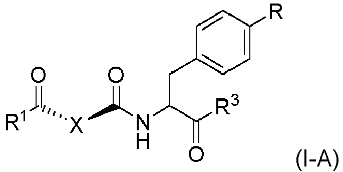 wherein X, R, R¹ and R³ are as defined in the present disclosure.

In a more preferred embodiment, the compound of formula (I-A) corresponds to the following enantiomer (I-A') : 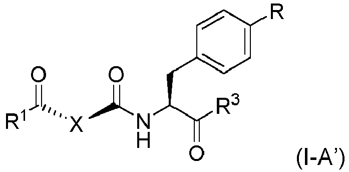 wherein X, R, R¹ and R³ are as defined in the present disclosure.

In a preferred embodiment, X is 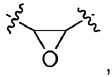 preferably

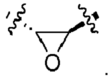

In another particular embodiment, X is -NH-CH₂-.

According to the invention, R is OR².

According to some embodiments, R² is a C₁-C₆ aliphatic chain, such as C₁-C₆ alkyl, or C₁-C₆ alkyl-aryl, said aliphatic chain or alkyl-aryl being optionally substituted. When R² is a C₁-C₆ alkyl, such as methyl or ethyl, it is notably unsubstituted (non substituted) or substituted with a phenyl. More preferably, R² is C₁-C₆ alkyl such as ethyl, or C₁-C₆ alkyl-aryl such as benzyl.

According to some other embodiments, R³ is OH, or O-C₁-C₆ aliphatic chain. More preferably, R³ is OH or O-C₁-C₆ alkyl, in particular O-ethyl, O-isopropyl or O-terbutyl.

According to some embodiments, R¹ is NR^{1a}R^{1b}, with R^{1a} being H and R^{1b} being C₁-C₆ alkyl, said alkyl being optionally substituted with C(O)-O-C₁-C₆ alkyl or C(O)-NH-C₁-C₆ alkyl . In such embodiments, R¹ may notably be NH-C₁-C₆ alkyl, said alkyl, such as a methyl, an ethyl, a n-propyl , an iso-propyl, a n-butyl or a t-butyl, being substituted with C(O)-O-C₁-C₆ alkyl or C(O)-NH-C₁-C₆ alkyl, notably with C(O)-methyl, C(O)-ethyl, C(O)-isopropyl or C(O)-terbutyl, or C(O)-NH-methyl, C(O)-NH-ethyl, C(O)-NH-isopropyl or C(O)-NH-terbutyl.

According to a preferred embodiment, R¹ is 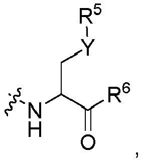 preferably

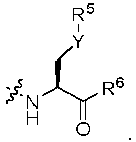

The compound of formula (I) may be a compound of the following formula : 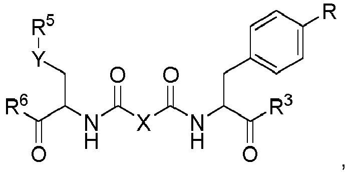 in which R, R³, R⁵, R⁶, X and Y are as defined in the present disclosure.

Preferably, the compound of formula (I) is a compound of formula (I-Aₐ) having the following configuration : 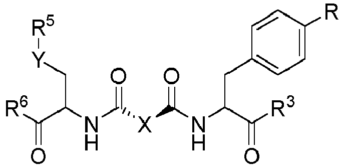 (I-Aₐ), in which R, R³, R⁵, R⁶, X and Y are as defined in the present disclosure.

More preferably, the compound of formula (I) is a compound of formula (I-A'ₐ) having the following configuration : 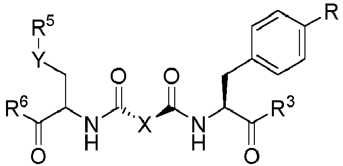 (I-A'a), in which R, R³, R⁵, R⁶, X and Y are as defined in the present disclosure.

Y may notably be 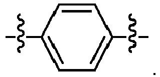 In such an embodiment, R⁵ is preferably O-C₁-C₆ alkyl, such as O-methyl or O-ethyl, notably O-ethyl.

Alternatively, Y may be -(CH₂)ₘ-, with m being an integer from 0 to 6, preferably m is 1 or 2, more preferably m is 2. In the embodiment wherein Y is -(CH₂)ₘ, R⁵ is preferably C(O)OH.

According to some embodiments, R⁶ is O-C₁-C₆ aliphatic chain, such as O-methyl or O-ethyl notably O-ethyl, in particular when Y is

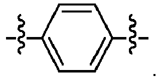

According to other specific embodiments, R⁶ is NH-CH(R⁷)-(CH₂)ₙ-R⁸, in particular when Y is - (CH₂)ₘ-, notably is -(CH₂)₂-. In such embodiments, R⁷ is preferably H and R⁸ is preferably aryl, such as phenyl and n is 1, 2 or 3, preferably 1.

According to a preferred embodiment, when R³ is OH, R¹ is 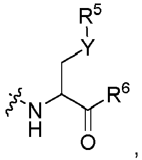 preferably

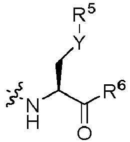

In a preferred embodiment, the compound of formula (I) is a compound of formula (I-A'a) as defined above, in which X, R and R³ are as defined above, R¹ is 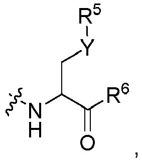 preferably 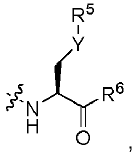 with:
- Y being -(CH₂)ₘ-, m being as defined above and notably m is 2, R⁵ being C(O)OH, R⁶ being NH-CH(R⁷)-(CH₂)ₙ-R⁸, R⁷ being H, R⁸ being aryl, such as phenyl and n being 1, 2 or 3, or
- Y being 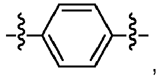 R⁵ being O-C₁-C₆ alkyl, such as O-methyl or O-ethyl, and R⁶ being O-C₁-C₆ aliphatic chain, such as O-methyl, or O-ethyl.

In another more preferred embodiment, the compound of formula (I) is of formula (I-A') in which X is 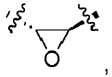 R¹ is NR^{1a}R^{1b}, with R^{1a} being preferably H and R^{1b} being preferably C₁-C₆ alkyl, such as a methyl, an ethyl, a n-propyl, a n-butyl or a t-butyl, said alkyl being substituted with C(O)-methyl, C(O)-ethyl, C(O)-isopropyl or C(O)-terbutyl, or C(O)-NH-methyl, C(O)-NH-ethyl, C(O)-NH-isopropyl or C(O)-NH-terbutyl, R is OR² with R² being preferably C₁-C₆ alkyl, in particular ethyl or C₁-C₆ alkyl, in particular benzyl, and R³ is OH or O-C₁-C₆ alkyl, in particular O-ethyl, O-isopropyl or O-terbutyl.

In another more preferred embodiment, the compound of formula (I) is of formula (I-A') in which X is 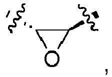 R¹ is NR^{1a}R^{1b}, with R^{1a} being preferably H and R^{1b} being preferably C₁-C₆ alkyl, such as a methyl, an ethyl, a n-propyl, an iso-propyl, a n-butyl or a t-butyl (ter-butyl), preferably an ethyl, an iso-propyl or a ter-butyl, R is OR² with R² being preferably C₁-C₆ aliphatic chain substituted by a phenyl, and R³ is O-C₁-C₆ alkyl, in particular O-ethyl, O-isopropyl or O-terbutyl.

In a particular embodiment, when X is 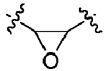
R¹ is preferably 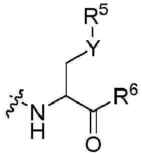 or NR^{1a}R^{1b} with R^{1a} is H and R^{1b} is C₁- C₆ alkyl non substituted or substituted with C(O)-O-C₁-C₆ alkyl or C(O)-NH-C₁-C₆ alkyl,
R is O-R² with R² is a C₁-C₆ aliphatic chain, said aliphatic chain being non substituted or substituted with a phenyl, or C₁-C₆ alkyl-aryl,
R³ is OH or O-C₁-C₆ aliphatic chain,
Y is -(CH₂)ₘ- with m=2 or 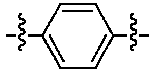
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain.

In some preferred embodiments,
R1 is 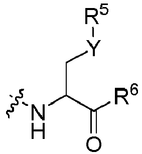 and
R is O-R² with R² is a C₁-C₆ aliphatic chain, said aliphatic chain being non substituted or substituted with a phenyl,
R³ is OH or O-C₁-C₆ aliphatic chain,
Y is -(CH₂)ₘ- with m=2 or 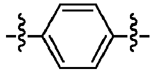
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain.

In another particular embodiment, when X= -NH-CH₂-
R¹ is preferably NR^{1a}R^{1b} with R^{1a} is H and R^{1b} is C₁- C₆ alkyl non substituted or substituted with C(O)-NH-C₁-C₆ alkyl,
R is O-R² with R² is C₁-C₆ alkyl-aryl, and
R³ is O-C₁-C₆ aliphatic chain.

In a particular embodiment, the compound of formula (I) according to the invention is a prodrug as defined above, notably selected from the following compounds: 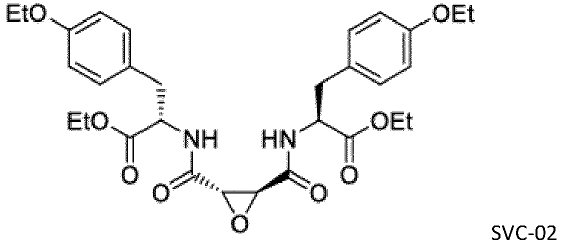 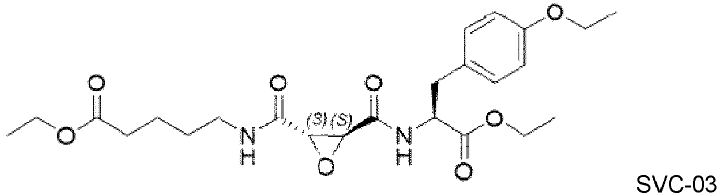 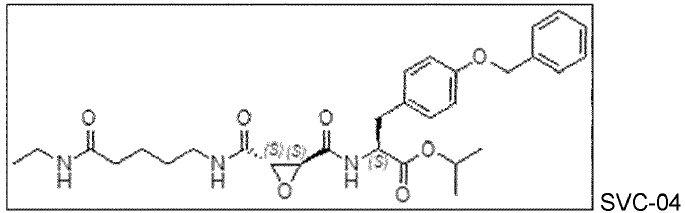 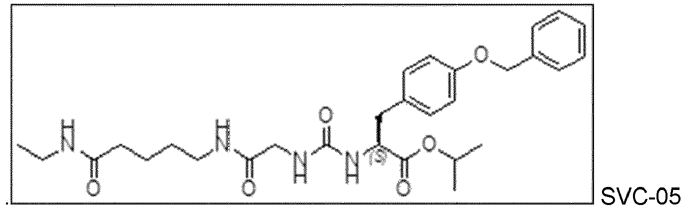 And

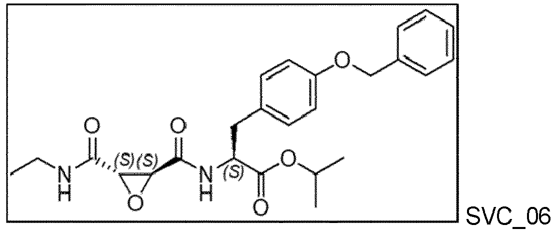

In a particular embodiment, the compound of formula (I) according to the invention is a drug as defined above, notably the following compound:

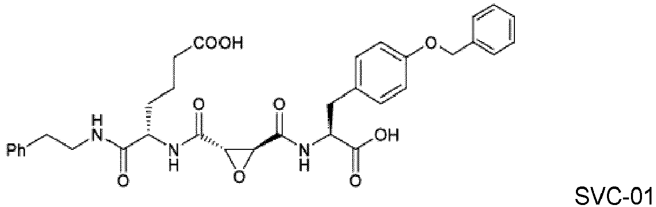

In a particular and preferred embodiment, the compound of formula (I) according to the invention is selected from the group consisting of the following compounds 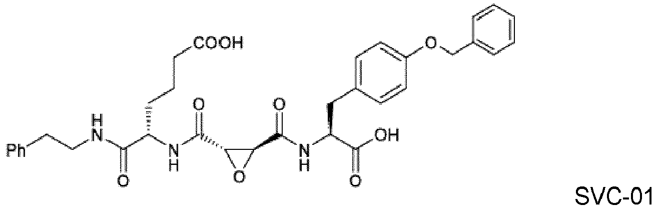 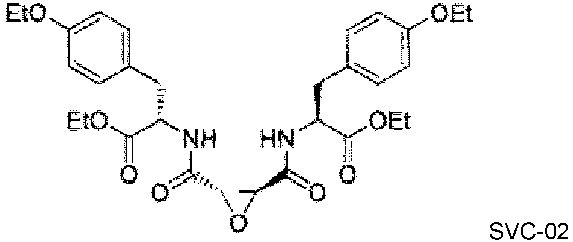 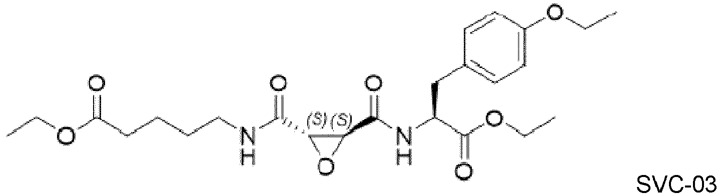 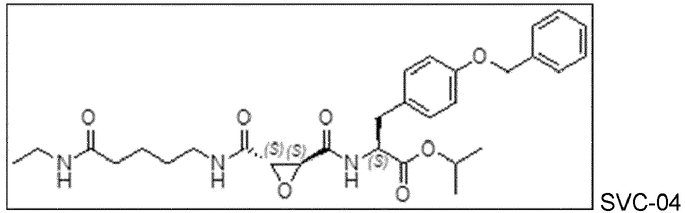 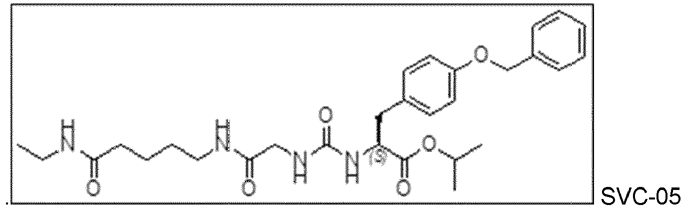 And 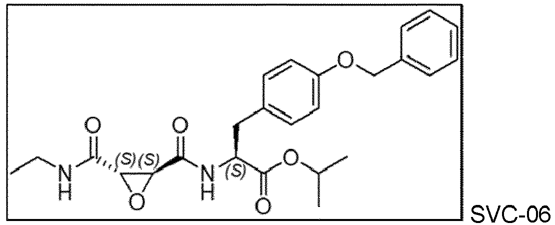 or conjugates thereof.

### Method of preparation of a compound of formula (I)

The compound of formula (I) as described above, or a pharmaceutically acceptable salt and/or solvate thereof, may be obtained by a method comprising the following steps:
(a) reacting a compound of formula (II) : 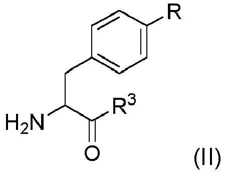
   with a compound of formula (III) : 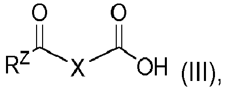
   wherein R^{Z} is R¹ as defined above or OH,
(b) optionally, converting R^{Z} being OH to R¹ as defined above.

The compound of formula (III) may be obtained according to methods well-known from the skilled person in the art.

The reaction between compound of formula (II) and formula (III) is notably a peptide coupling as defined above.

The compound of formula (II) is notably

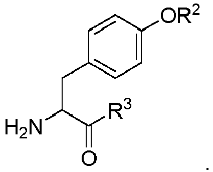

Optionally, additional steps of protection/deprotection and/or of functionalization well-known from the skilled person in the art may occur before or after the reaction between compounds of formula (II) and (III) to afford compound of formula (I) with the suitable substituents as described above. In particular, when R^{Z} is OH, the compound resulting from step (a) undergoes a peptide coupling with 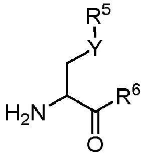 to provide a compound of formula (I) in which R¹ is

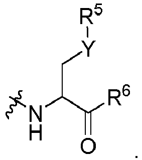

The one or more peptide coupling carried out in the method of preparation of compound of formula (I) are notably achieved in presence of PyAOP as coupling agent. Preferably, the base DIEA is also used.

The peptide coupling may be carried out on a solid support, notably by using a resin to which one of the reagents, the amine part or the acid part, is attached. Such methods are well-known from the skilled person in the art.

### Conjugate comprising a fragment of a compound of formula (I) linked to a biomolecule

The present invention also relates to a use of a conjugate comprising a fragment of a compound of formula (I) as described above linked to a biomolecule such as a peptide, a protein, a biomarker, as for example a photolabelling agent, such as Rhodamine, cyanines derivatives or fluoresceine, an affinity probe such as biotin, or a E3 ubiquitin ligase recruiter including but not limited to Thalidomide, VH032, VH101, dBET1, dFKBP12, QCA570, ZNL-02-096 or d9A-2.

The term" fragment of a compound of formula (I)" refers to the compound of formula (I) in which one extremity is modified due to the bonding to a biomolecule, for example via a linker. Typically, the R¹ group is thus modified for allowing said bonding. For instance, in a conjugate according to the invention, the fragment of a compound of formula (I) refers to the following moiety : 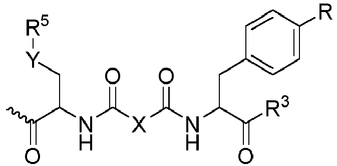 wherein 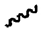 is the single bond between the fragment and the rest of the conjugate, and R, R³, X, Y and R⁵ are as defined in the present disclosure.

According to a particular embodiment, the conjugate according to the present invention has the following formula (I'): 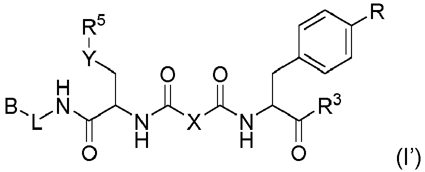
or a pharmaceutically acceptable salt and/or solvate thereof,
wherein
B is a biomolecule such as a peptide, a protein, a biomarker, as for example a photolabelling agent, or a E3 ligase recruiter such as Thalidomide,
L is a linker, and
R, R³, X, Y and R⁵ are as defined above.

According to a preferred embodiment, the conjugate of formula (I') is a conjugate of formula (I'-A) having the following configuration:

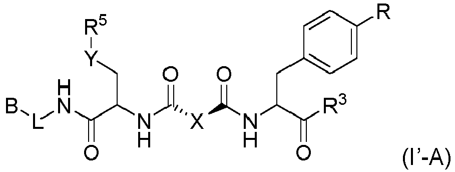

In particular, the conjugate of formula (I') is a conjugate of formula (I'-Aₐ) having the following configuration :

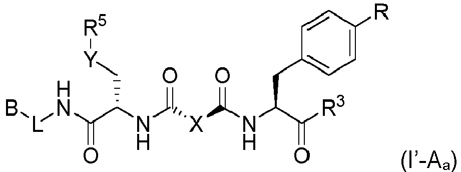

In the conjugate of formula (I'), in particular of formula (I'-Aa), X is preferably

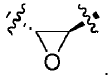

R is preferably OR², R² being advantageously C₁-C₆ alkyl, in particular ethyl, or C₁-C₆ alkyl-aryl, in particular benzyl.

R³ is preferably OH or O-C₁-C₆ alkyl, in particular O-ethyl, O-isopropyl or O-terbutyl.

Y may be -(CH₂)ₘ- or

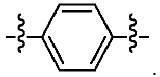

When Y is -(CH₂)ₘ-, in particular -(CH₂)₂-, R⁵ is preferably C(O)OH, R⁶ is preferably NH-CH₂-(CH₂)ₙ-R⁸, with R⁸ being advantageously aryl, such as phenyl, and n being advantageously 1, 2 or 3, notably 1.

When Y is 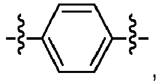 R⁵ is preferably O-C₁-C₆ alkyl, such as O-ethyl.

According to some embodiments, the linker L corresponds to a divalent radical derived from a C₁-C₁₂ aliphatic chain, wherein one or more methylene unit(s) are replaced by structural linkers selected from arylene or fragments -O-, -S-, -C(=O)-, -SO₂- or -N(C₁-C₆ alkyl)-, wherein said aliphatic chain is unsubstituted or is substituted with one or more radicals selected from halogen, OH, a C₁-C₆ alkyl and/or a C₁-C₆ alkyl aryl group, such as benzyl group.

In an embodiment, the biomolecule is an affinity probe and the conjugate of formula (I') may be : 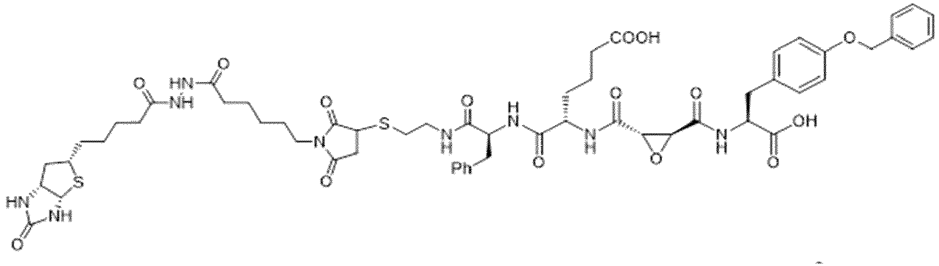 in which a compound of formula (I) is linked to biotin.

In another embodiment, the biomolecule is a photolabeling agent and the conjugate of formula (I') may be : 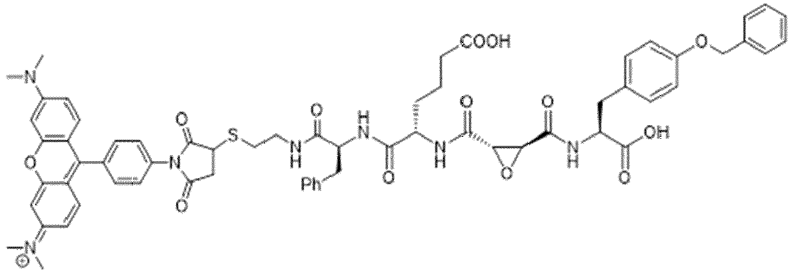 in which a compound of formula (I) is linked to Rhodamine.

### Pharmaceutical composition used in the present invention

The present invention also relates to a pharmaceutical composition used in the prevention or treatment of heart disorders, comprising at least one pharmaceutically acceptable excipient and at least one compound of formula (I) as described above or a pharmaceutically acceptable salt and/or solvate thereof.

The present invention also relates to a pharmaceutical composition used in the prevention or treatment of heart disorders, comprising at least one conjugate as described above, such as a conjugate of formula (I'), or a pharmaceutically acceptable salt and/or solvate thereof, and at least one pharmaceutically acceptable excipient.

In some embodiments, the pharmaceutical compositions used according to the invention is intended to oral or parenteral (including but not limited to intravenous, intramuscular, intra-arterial, intradermal, subcutaneous, intraperitoneal, intraventricular, and intracranial), preferably oral or intravenous administration. Other modes of delivery include, but are not limited to, the use of liposomal formulations, intravenous infusion, transdermal patches, etc. The active ingredient can be administered in unit forms for administration, mixed with conventional pharmaceutical carriers, to animals, preferably mammals including humans.

These compositions can be delivered transdermally, by a topical route, formulated as applicator sticks, solutions, suspensions, emulsions, gels, creams, ointments, pastes, jellies, paints, powders, and aerosols. Oral preparations include tablets, pills, powder, capsules, liquids, lozenges, cachets, gels, slurries, suspensions, etc., suitable for ingestion by the patient. Solid form preparations include powders, tablets, pills, capsules, cachets, suppositories, and dispersible granules. Liquid form preparations include solutions, suspensions, and emulsions, for example, water or water/propylene glycol solutions. The compositions may additionally include components to provide sustained release and/or comfort. Such components include high molecular weight, anionic mucomimetic polymers, gelling polysaccharides and finely-divided drug carrier substrates. The compositions can also be delivered as microspheres for slow release in the body. For example, microspheres can be administered via intradermal injection of drug-containing microspheres, which slowly release subcutaneously ; as biodegradable and injectable gel formulations; or, as micro spheres for oral administration. In another embodiment, the compositions can be delivered by the use of liposomes which fuse with the cellular membrane or are endocytosed, i.e., by employing receptor ligands attached to the liposome, that bind to surface membrane protein receptors of the cell resulting in endocytosis. By using liposomes, particularly where the liposome surface carries receptor ligands specific for target cells, or are otherwise preferentially directed to a specific organ (e.g., the heart), one can focus the delivery of the compositions into the target cells in vivo.

Co-administration is meant to include simultaneous or sequential administration of the compound individually or in combination (more than one compound or agent) with other active substances, such as the ones disclosed hereunder.

### Combination therapy or combination product

The compound of formula (I) can be combined with other current standard-of-care treatments for heart failure, cardiomyopathies, or myocardial infarction induced cardiac dysfunction, such as the ones listed in the 2021 ESC Guidelines for the diagnosis and treatment of acute and chronic heart failure (McDonagh T.A et al., 2021).

The specialist in heart failure will define the most appropriate combination depending on the subject and the heart disease. Combinations with an SVC compound (as a dual pharmacophore) might seem intuitively attractive:
1) Administration together with a positive inotrope that has a different and complementary mechanism of action (MoA). This could be a beta agonist (e.g. dobutamine, dopamine or epinephrine), a type 3 phosphodiesterase inhibitor (like milrinone) or an alternative positive inotrope like levosimenden to boost the total positive inotropic rescue strategy in a cardiogeneic shock state without increasing energy requirements.
2) Administration together with a negative inotrope that should be beneficial, but is poorly tolerated due to its negative inotropic action. This could be a combination with a beta-adrenergic blocker in a patient with severe cardiomyopathy or cardiogenic shock, a combination with a myosin inhibitor (like mavacamten or other emerging agents).

So, the present invention also relates to a method treating cardiovascular disease selected from heart failure, cardiomyopathies and myocardial infarction induced cardiac dysfunction in a subject in need thereof, comprising administering to the subject in need thereof of an effective dose of a compound of formula (I) as disclosed above in combination with a current standard-of-care treatment for heart failure, cardiomyopathies, or myocardial infarction induced cardiac dysfunction. In particular, the current standard-of-care treatment for heart failure is selected from a positive inotrope or a negative inotrope, in particular (i) a positive inotrope selected in the group consisting of a beta agonist, a type 3 phosphodiesterase inhibitor and an alternative positive inotrope, or (ii) a negative inotrope selected in the group consisting of a beta-adrenergic blocker and a myosin inhibitor.

Another object of the present invention is a combination product comprising:
a) A compound for formula (I) as defined in the present invention or a pharmaceutical composition as defined in the present invention, and
b) A current standard-of-care treatment for heart failure, cardiomyopathies, or myocardial infarction induced cardiac dysfunction.
In particular, the current standard-of-care treatment for heart failure is selected from a positive inotrope or a negative inotrope, in particular (i) a positive inotrope selected in the group consisting of a beta agonist, a type 3 phosphodiesterase inhibitor and an alternative positive inotrope, or (ii) a negative inotrope selected in the group consisting of a beta-adrenergic blocker and a myosin inhibitor.

### Uses of compounds and pharmaceutical compositions in heart disorders

The compound of formula (I), the pharmaceutically acceptable salt and/or solvate thereof, or the pharmaceutical composition according to the invention act as VASH inhibitors, meaning that it is able to inhibit the peptidase activity of VASH that catalyzes microtubules detyrosination. When this VASH peptidase activity is deregulated, and notably abnormally increased, it induces heart disorders and in particular heart failure, cardiomyopathies and/or myocardial infarction induced cardiac dysfunction.

The compounds of formula (I) in which X is 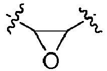 are notably irreversible VASH inhibitors, which covalently bind to the VASH enzymes.

The present invention relates to a compound of formula (I) according to the invention or a pharmaceutically acceptable salt and/or solvate thereof, for use as a drug in the prevention and/or the treatment of heart disorders and in particular heart failure, cardiomyopathies, and/or myocardial infarction induced cardiac dysfunction.

In other terms, the present invention relates to the use of a compound of formula (I) according to the invention or a pharmaceutically acceptable salt and/or solvate thereof, for the manufacture of a drug, intended in the prevention and/or the treatment of heart disorders and in particular heart failure, cardiomyopathies, and/or myocardial infarction induced cardiac dysfunction.

In other terms, the present invention relates to the use of a compound of formula (I) according to the invention or a pharmaceutically acceptable salt and/or solvate thereof for the prevention and/or the treatment of heart disorders and in particular heart failure, cardiomyopathies and/or myocardial infarction induced cardiac dysfunction.

In particular, the present invention relates to the use of a compound of formula (I) according to the invention or a pharmaceutically acceptable salt and/or solvate thereof for use in the prevention and/or the treatment of heart failure, cardiomyopathies, myocardial infarction induced cardiac dysfunction and/or for improving heart function in a subject in need thereof.

In other terms, the present invention relates to a method for improving heart function in a subject in need thereof, comprising administering to a subject in need thereof of a compound of formula (I) according to the invention or a pharmaceutically acceptable salt and/or solvate thereof.

In particular, the compound of formula (I) or a pharmaceutically acceptable salt and/or solvate thereof improves kinetics of failing cardiomyocytes including increasing relaxation speed, increasing contractility, and/or reducing viscoelasticity.

In other terms, the present invention relates to a method treating cardiovascular disease selected from heart failure, cardiomyopathies and myocardial infarction induced cardiac dysfunction in a subject in need thereof, comprising administering to a subject in need thereof of a compound of formula (I) according to the invention or a pharmaceutically acceptable salt and/or solvate thereof.

In other terms, the present invention relates to a method for the prevention and/or the treatment of heart disorders and in particular heart failure, cardiomyopathies and/or myocardial infarction induced cardiac dysfunction comprising the administration to a subject in need thereof of an effective dose of a compound of formula (I) according to the invention or a pharmaceutically acceptable salt and/or solvate thereof.

According to another aspect, the present invention relates to a pharmaceutical composition according to the invention for use as a drug in the prevention and/or the treatment of heart disorders and in particular heart failure, cardiomyopathies and/or myocardial infarction induced cardiac dysfunction.

In other terms, the present invention relates to the use of a pharmaceutical composition according to the invention for the manufacture of a drug intended in the prevention and/or the treatment of heart disorders and in particular heart failure, cardiomyopathies and/or myocardial infarction induced cardiac dysfunction.

In other terms, the present invention relates to the use of a pharmaceutical composition according to the invention for the prevention and/or the treatment of heart disorders and in particular heart failure, cardiomyopathies and/or myocardial infarction induced cardiac dysfunction.

In other terms, the present invention relates to a method for the prevention and/or the treatment of heart disorders and in particular heart failure, cardiomyopathies and/or myocardial infarction induced cardiac dysfunction, comprising the administration to a subject in need thereof of an effective dose of a pharmaceutical composition according to the invention.

In certain embodiments, the subject in need thereof has heart failure associated with failing cardiomyoctes.

In certain embodiments, the subject in need thereof has heart failure with reduced ejection fraction (HFrEF).

In certain embodiments, the subject in need thereof has heart failure with preserved ejection fraction (HFpEF).

In certain embodiments, the pharmaceutical composition of the invention is used in reducing viscoelasticity and/or speed up cardiomyocyte relaxation.

In some embodiments, the pharmaceutical composition of the invention is used in increasing the speed of cardiomyocyte relaxation via a Ca²⁺ independent mechanism.

In certain embodiments, the pharmaceutical composition of the invention is used for reducing cardiomyocyte stiffness, improving myocyte relaxation, increasing ventricular compliance, and/or reducing diastolic pressures.

In certain embodiments, the pharmaceutical composition of the invention is used in treating patients with diastolic dysfunction.

In certain embodiments, the pharmaceutical composition of the invention is used in treating chronic heart disorders, in particular chronic heart failure or cardiomyopathies.

In certain embodiments, the pharmaceutical composition of the invention is used in treating acute heart failure (AHF) syndromes including post-myocardial infarction, post-cardiotomy states, following cardiac arrest, in the setting of hypertensive crises, acute presentations of non-ischemic cardiomyopathies, and acute exacerbations of chronic cardiomyopathies of various etiologies.

In certain embodiments, the pharmaceutical composition of the invention is used in treating patients with systolic dysfunction.

In certain embodiments, the pharmaceutical composition of the invention is used in preparing a medicament for use in treatment of failing cardiomyocytes.

In certain embodiments, the pharmaceutical composition of the invention is used in preparing a medicament for use in improvement of kinetics of failing ventricular cardiomyocytes.

In certain embodiments, the pharmaceutical composition of the invention is used in preparing a medicament for use in reducing viscoelasticity of cardiomyocytes.

### Research tools uses

Another aspect of the present invention is a use of the compound (I) as defined in the present invention or a conjugate comprising a fragment of a compound of formula (I) linked or not to a biomolecule optionally labeled, as research tool for research and development activities in heart function disorders or heart failure.

In some embodiments, the present invention relates to a compound of formula (I) according to the invention or a conjugate thereof for use as research tool for research and development activities selected in the group consisting of:
- *in vitro* method for studying the role of tubulin detyrosination in heart disorders, such as its occurrence, aggressiveness and progression,
- and related kits for performing said screening assays and methods.

These R&D activities as examples of illustrative but not limitative uses of said compounds for formula (I) or conjugate thereof.

The said compounds for formula (I) may be coupled for example but not limited to fluorescent dyes, UV-sensitive dyes, HRP, alkaline phosphatase, biotin.

### Description of the figures

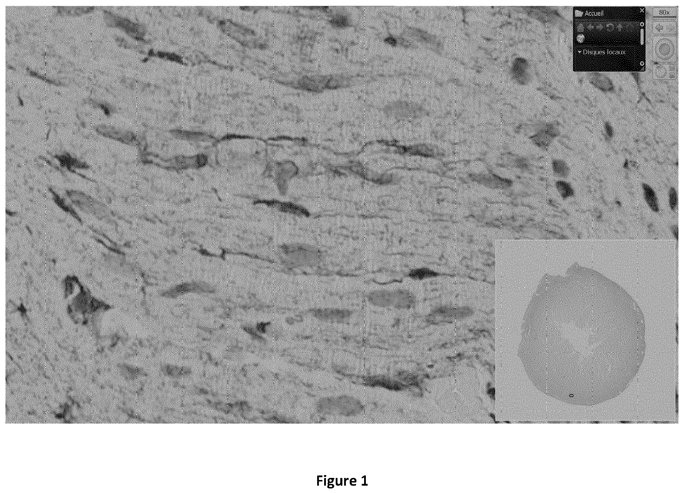

**Figure** 1: High magnification image of immunohistochemistry staining of detyrosinated tubulin on healthy myocardial tissue. High magnification showing single cardiomyocytes. Purple stain is specific detyrosinated tubulin.

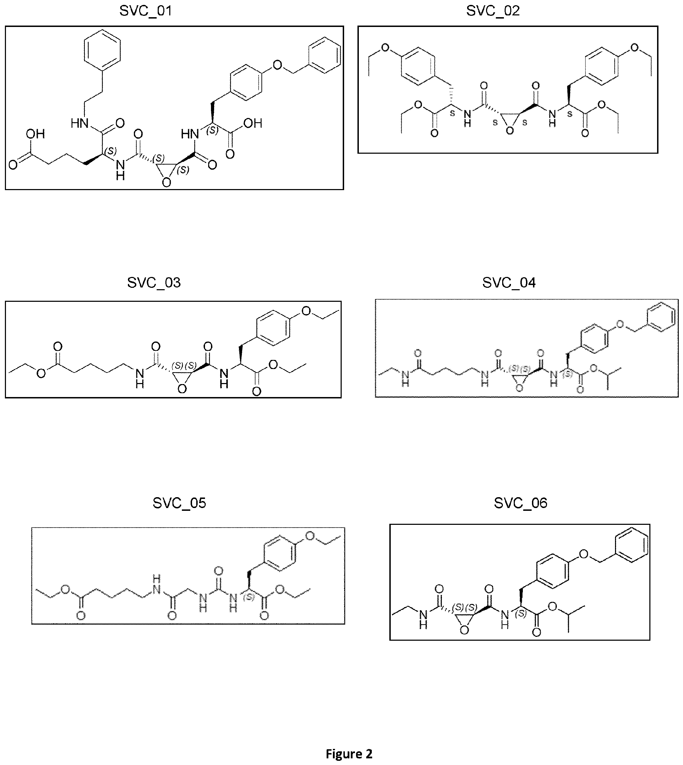

**Figure 2****:** Structure of Specific Vash Compounds (SVC) that act as potent inhibitors.

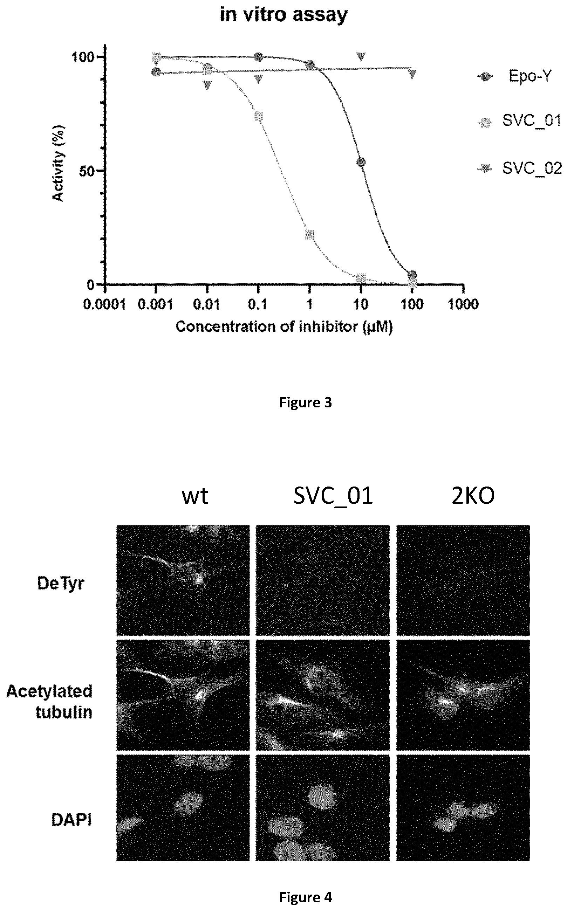

**Figure 3****:** In vitro detyrosination assays using recombinant human VASH1 (hVASH1) and SVBP complex. ELISA-based method to measure detyrosinase activity. Dose-response allows for accurate determination of the inhibition of the compounds on the target. Of note, SVC_02 is a prodrug. The compound is inactive in its prodrug form when tested using recombinant enzyme. It contains a specific group that effectively mask a second group essential for binding the enzyme.
**Figure 4****:** Immuofluorescent staining of wild type human cells and VASH double knock-outs. SVC_01 treatment leads to background signal comparable to that observed in VASH 2KO cells demonstrating entire target engagement. The levels of acetylated tubulin remain unaffected by SVC_01 treatment as highly similar staining patterns and levels are observed in the different conditions.

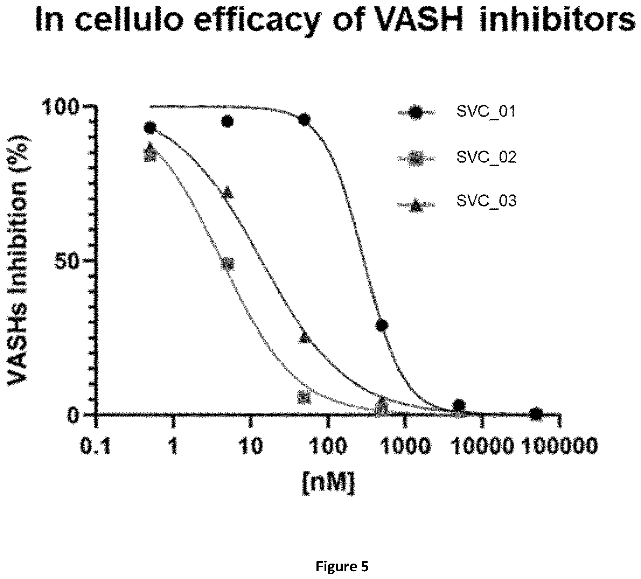

**Figure 5****:** Immunofluorescent method to detect levels of tubulin detyrosination in human cells. Dose dependent reduction of tubulin detyrosination in human cells is observed after 2 hours treatment with all SVCs. In cellulo IC50 measure for SVC_02 = 10nM.

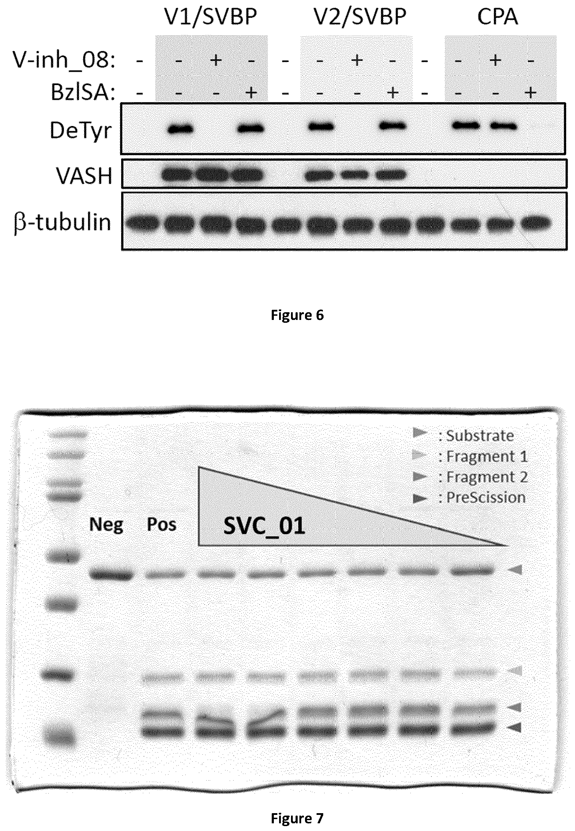

**Figure 6****:** In vitro detyrosination assays using recombinant human enzymes in absence or presence of SVC_01 or BzlSA (specific carboxypeptidase A inhibitor). Carboxypeptidase A (CPA) hydrolyzes peptide bonds of C-terminal residues with aromatic or aliphatic side-chains No cross reactions observed between the different enzymes. Reactions were stopped using SDS-page laemli buffer and loaded for western blotting using specific antibodies.
**Figure 7****:** Assay using active recombinant Human rhinovirus (HRV) type 14 3C protease Cysteine in absence (neg) or presence (increasing dose) of SVC_01. HRV is a protease with preference for non-aromatic residues at P1'. The optimum recognition site for Human rhinovirus (HRV) type 14 3C protease is LEVLFQ/GP, with a glycine at the P1' position. SVC_01 does not reduce activity of this cysteine protease.

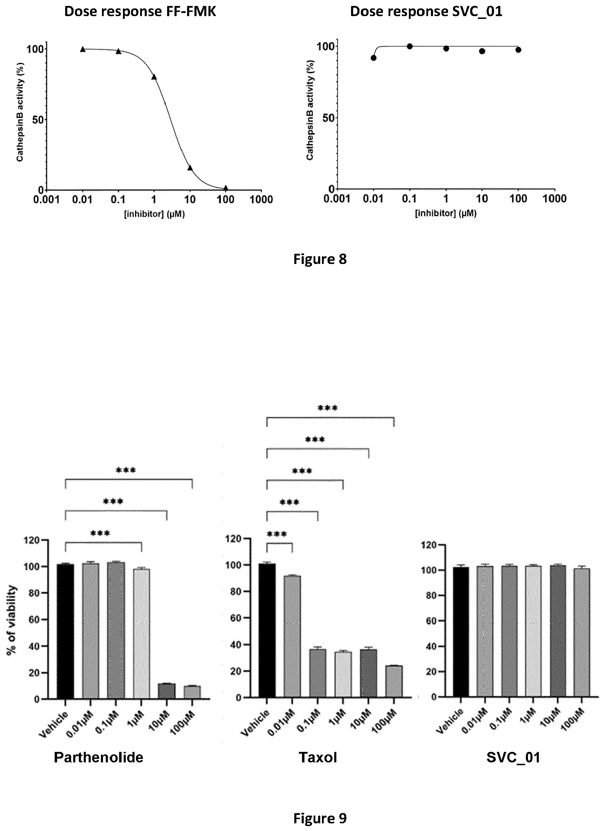

**Figure 8****:** Assay using active Cathepsin B Cysteine protease. The panels show activity of Cathepsin B in presence of commercial Cathepsin B&L inhibitor termed FF-FMK (positive control) or in presence of increasing dose of SVC_01. Cathepsin B is a cysteine protease with preference for aromatic residues at P1' site. Alike VASH, cathepsin B prefers an aromatic residue at the P1' position. This is considered to be our best control for specificity.
**Figure 9****:** Cell viability assay comparing microtubule binding drugs (Parthenolide, Taxol) to SVC_01 exposure on human cells. The MTT assay is used to measure cellular metabolic activity as an indicator of cell viability, proliferation and cytotoxicity. This colorimetric assay is based on the reduction of a yellow tetrazolium salt (3-(4,5-dimethylthiazol-2-yl)-2,5-diphenyltetrazolium bromide or MTT) to purple formazan crystals by metabolically active cells. As opposed to the microtubule binding drugs that display severe toxicity, SVC_01 does not impact cell proliferation.

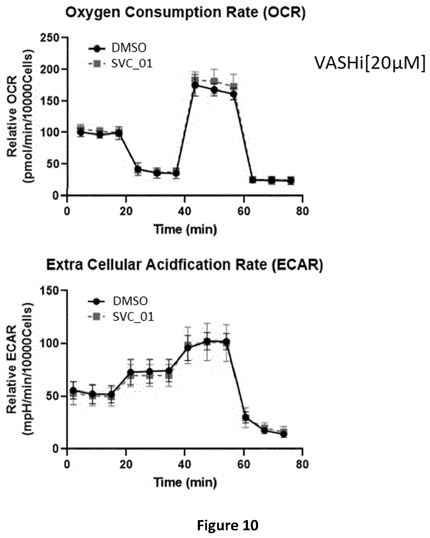

**Figure 10****:** Mitochondrial activity assays using the Agilent Seahorse Cell Mito Stress Test kit in absence or presence of SVC_01 [20µM]. This assay is the widely recognized, well-accepted standard assay for assessing energetic mitochondrial function. Multiple parameters are obtained in this one assay including, basal respiration, ATP-linked respiration, maximal and reserve capacities, and non-mitochondrial respiration. The assay provides insights into the mechanism of mitochondrial dysfunction and allow users to investigate functional differences among cell types, drug candidates, and genetic or biochemical interventions. High exposure to SVC_01 does not alter mitochondrial activity.

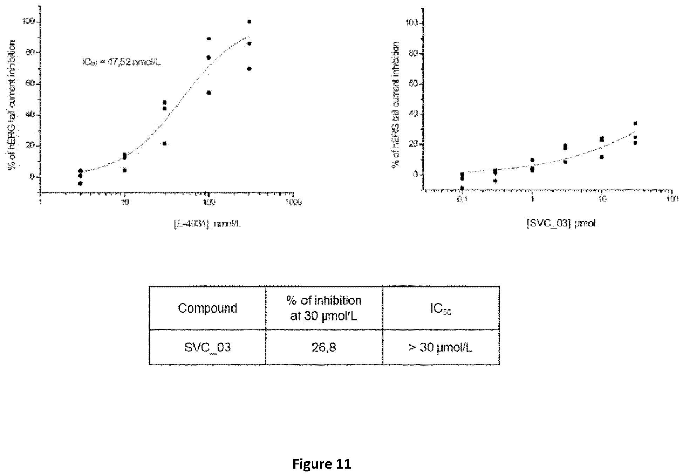

**Figure 11****:** Potassium currents mediated by hERG channels (called hERG current) were recorded with a patch-clamp technique using the whole cell configuration. The percent of inhibition values represent the mean of percentages, calculated from individual variation of hERG tail current amplitude for each cell vs extracellular solution (stabilization phase). The mean of percent of inhibition at the top test concentration i.e.at 30 µmol/L is reported in Table. For each compound, a 6 points concentration-response curve was established.

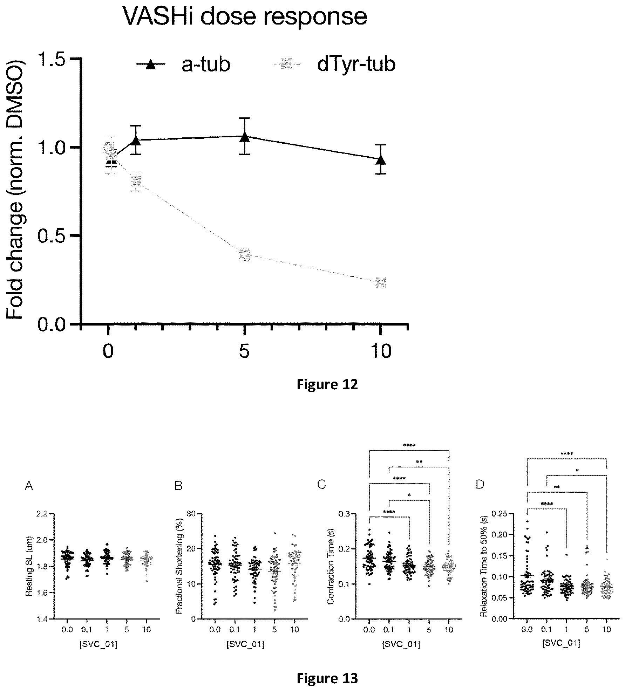

**Figure 12****:** Dose-dependent reduction in detyrosination upon VASH inhibition in primary cardiomyocytes isolated from Sprague Dawley rats. A) Representative western blot and B) quantification of detyrosinated and alpha-tubulin levels after 2hrs 37°C incubation of freshly isolated adult rat cardiomyocytes with compound SVC_01 at indicated concentrations. N = 4 Sprague Dawley rats.
**Figure 13****:** Dose-dependent modest improvement in contraction and relaxation kinetics during unloaded shortening upon VASH inhibition in primary cardiomyocytes isolated from healthy Sprague Dawley rats. A) Resting sarcomere length, B) fractional shortening, C) contraction time and D) relaxation time in healthy isolated rat cardiomyocytes. n = 50-60 myocytes per group from N = 3 Sprague Dawley rats. One-way ANOVA.

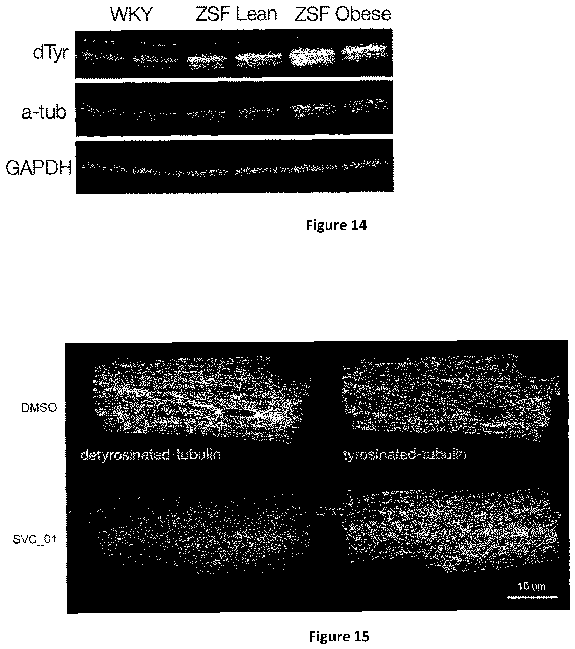

**Figure 14****:** Western blot from myocardial tissue prepared from Wistar Kyoto (WKY), ZSF1 lean and ZSF1 obese rats. Protein samples were prepared and analysed by immunoblotting following PAGE separation using mentioned specific antibodies.
**Figure 15****:** Immunofluorescence image of detyrosinated and tyrosinated tubulin in ZSF-1 Obese cardiomyocyte +/- 2hr 37C SVC_01 incubation shows virtual elimination of detyrosinated microtubule with maintained tyrosinated microtubule network.

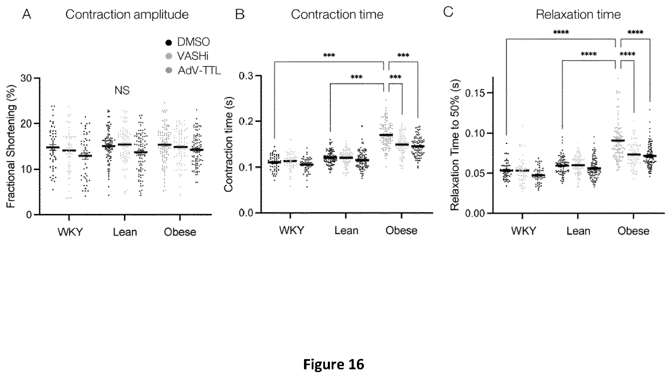

**Figure 16****:** Improved contraction and relaxation kinetics of HFpEF myocytes during unloaded shortening upon VASH inhibition or TTL overexpression in primary cardiomyocytes isolated from WKY, ZSF1-Lean, or ZSF1 Obese rats. A) Contraction amplitude, B) contraction time, and C) relaxation time in isolated rat cardiomyocytes. n = ~90 myocytes per group from N = 3 ZSF1 Lean or Obese rats; n = 60 myocytes from N = 2 WKY rats. Two-way ANOVA with factors of genotype and treatment.

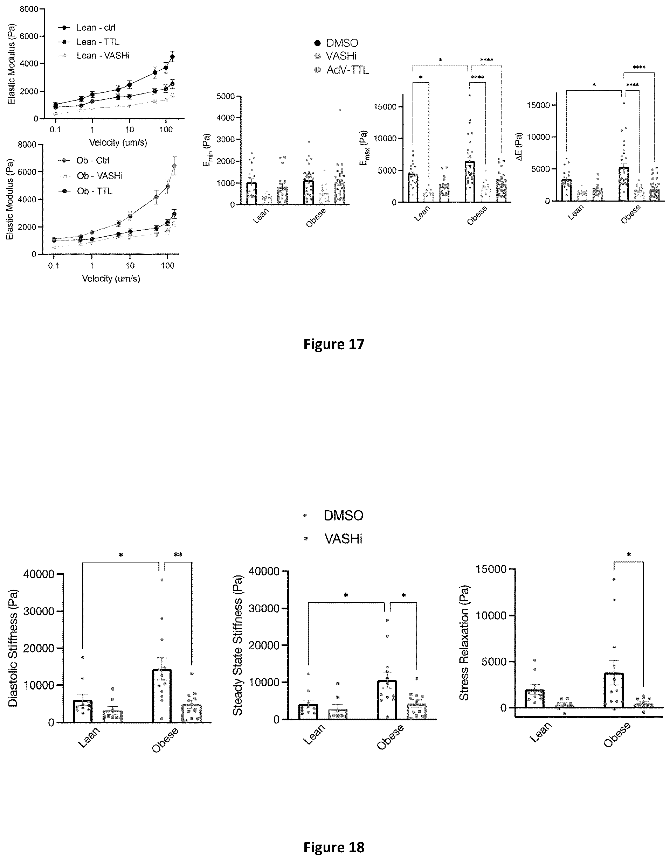

**Figure 17****:** Reduced transverse stiffness upon VASH inhibition or TTL overexpression in primary cardiomyocytes isolated from ZSF1-Lean or ZSF1 Obese rats. A) Stiffness (elastic modulus) plotted as a function of nanoindentation velocity in ZSF1-Lean and B) ZSF1-Obese myocytes. C) Quantification of Emin (elastic stiffness), Emax (stiffness at maximal velocity of indentation) and D) DeltaE (indicator of viscoelasticity) in ZSF1-Lean and Obese cardiomyocytes. ZSF1 Obese cardiomyocytes are more viscoelastic than their lean counterparts, and this viscoelastic stiffness is reduced by SVC_01 and TTL overexpression.
**Figure 18****:** Reduced longitudinal stiffness upon VASH inhibition in primary cardiomyocytes isolated from ZSF1-Lean or ZSF1 Obese rats. A) Diastolic Stiffness (elastic modulus at the initial time of peak strain) upon a 10%, 200ms strain along the long-axis of an isolated cardiomyocyte; B) Stead state stiffness upon strain and hold, and C) stress relaxation (indicator of viscoelasticity) in ZSF1-Lean and Obese cardiomyocytes. ZSF1 Obese cardiomyocytes are more viscoelastic than their lean counterparts, and this viscoelastic longitudinal stiffness is reduced by SVC_01 treatment.

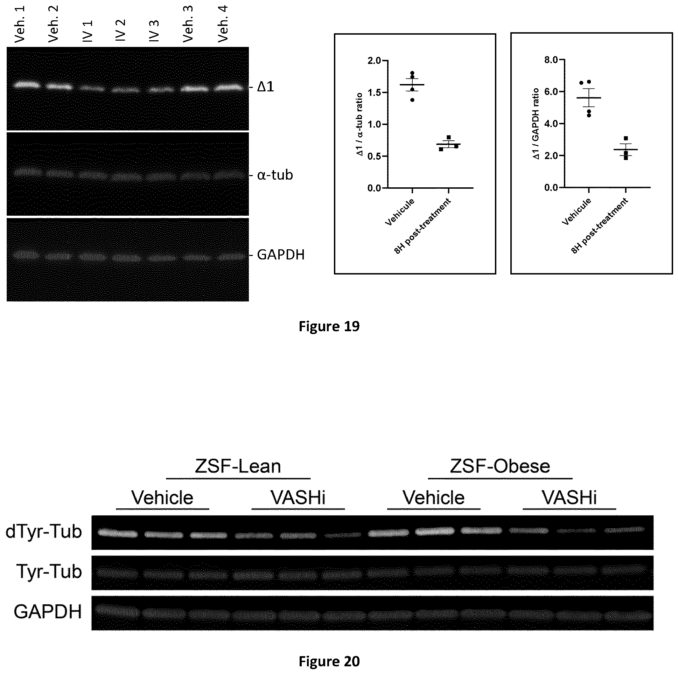

**Figure 19****:** A) Western blot from myocardial tissue demonstrating efficient reduction in detyrosinated tubulin upon single injection of SVC_02 into 8 weeks old Sprague Dawley rats. B) Quantification of detyrosination levels (D1/alpha-tubulin ratio) in vehicle (n=4) and VASHi-treated rats (n=3).
**Figure 20****:** Western blot from myocardial tissue demonstrating efficient reduction in detyrosinated tubulin upon two IV injections of SVC_02 into 30 weeks old ZSF1-Lean and Obese rats.

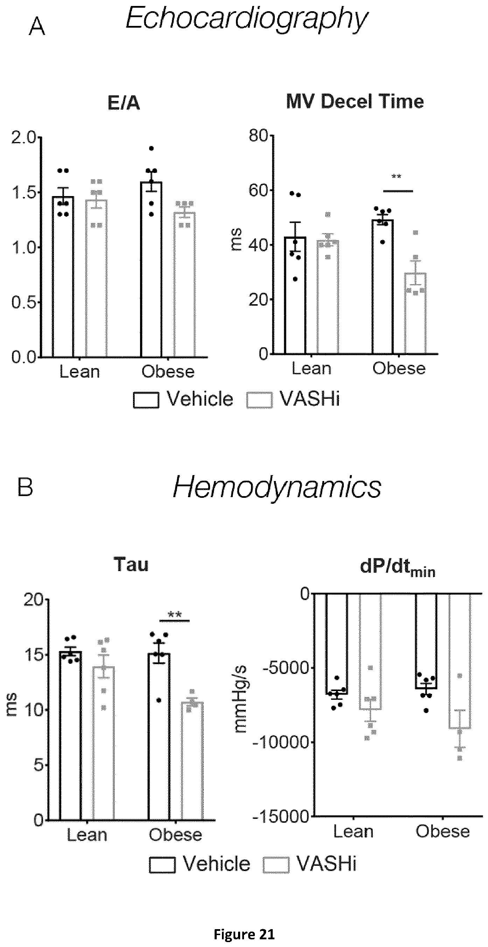

**Figure 21****:** VASHi acutely improves diastolic function in HFpEF rats. A) Echocardiographic assessments of left ventricular diastolic function in ZSF1 Lean and Obese animals +/- SVC_02 treatment. Improved E/A ratio (marker of the function of the left ventricle of the heart) and mitral valve deceleration time indicate improved diastolic function and faster ventricular relaxation upon VASH inhibition in ZSF1 obese rats. B) Hemodynamic measurements of left ventricular diastolic function in ZSF1 Lean and Obese animals +/- SVC_02 treatment. Improved tau (left ventricular diastolic time constant) and dP/dTmin (rate of ventricular relaxation) indicate more rapid ventricular relaxation upon VASH inhibition in ZSF1 obese rats.

### EXAMPLES

### Materials and Methods

### Animals

Animal care and procedures were approved and performed in accordance with the standards set forth by the University of Pennsylvania Institutional Animal Care and Use Committee and the Guide for the Care and Use of Laboratory Animals published by the US National Institutes of Health.

### Rat cardiomyocyte isolation and culture

Primary adult ventricular cardiac myocytes were isolated from 6 to 8 weeks old Sprague Dawley rats or 20 to 30 weeks old WKY, ZSF1 lean and obese rats. Briefly, the heart was removed from a rat anesthetized under isoflurane and retrograde-perfused on a Langendorff apparatus with a collagenase solution. The digested heart was then minced and triturated using a glass pipette. The resulting supernatant was separated and centrifuged at 300 revolution per minute (rpm) to isolate cardiomyocytes which were then resuspended in rat cardiomyocyte media at low density. Cardiomyocytes were cultured at 37°C and 5% CO₂ with 25 µmol/L of cytochalasin D. Rat cardiomyocyte media: medium 199 (Thermo Fisher 115090) supplemented with 1x Insulin-transferrin-selenium-X (Gibco 51500056), 1 µg µl⁻¹ primocin (Invivogen ant-pm-1), 20 mmol/L HEPES at pH 7.4 and 25 µmol/L cytochalasin D.

### Isolated cardiomyocyte contractility

Contractility was measured in a custom-fabricated cell chamber (lonOptix) mounted on an LSM Zeiss 880 inverted confocal microscope using either a 40 or 63 X oil 1.4 numerical aperture objective and transmitted light camera (lonOptix MyoCam-S). Experiments were conducted at room temperature and field stimulation was provided at 1.0 Hz with a cell stimulator (MyoPacer, IonOptix). After 10-30 seconds of pacing to achieve steady state, five traces were recorded and analyzed. Sarcomere length was measured optically by Fourier transform analysis (lonWizard, IonOptix).

### Western blot

For whole cell protein extraction, isolated rat cardiomyocytes were lysed in RIPA buffer (Cayman #10010263) supplemented with protease and phosphatase Inhibitor cocktail (Cell Signaling #5872S) on ice for 1 hour. The supernatant was collected and combined with 4X loading dye (Li-COR #928-40004), supplemented with 10% 2-mercaptoethonol, and boiled for 8 minutes. The resulting lysate was resolved on SDS-PAGE gel and protein was blotted to nitrocellulose membrane (Li-COR #926-31902) with miniTrans-Blot Cell (Bio-Rad). Membranes were blocked for an hour in Odyssey Blocking Buffer (TBS) (LI-COR #927-50000) and probed with corresponding primary antibodies overnight at 4 °C. Membranes were rinsed with TBS containing 0.5% Tween 20 (TBST) three times and incubated with secondary antibodies TBS supplemented with extra 0.2% Tween 20 for 1 hour at room temperature. Membranes were washed again with TBST (0.5% Tween 20) and imaged on an Odyssey Imager. Image analysis was performed using Image Studio Lite software (LI-COR). All samples were run in duplicates and analyzed in reference to GAPDH.

### Antibodies

Detyrosinated tubulin; rabbit polyclonal (Abcam ab48389); or affinity purified antibody that specifically detects detyrosinated alpha tubulin (Van der Laan, 2019); western blot: 1: 1,000. Alpha tubulin; mouse monoclonal, clone DM1A (Cell Signaling #3873); western blot: 1:1,000. GAPDH; mouse monoclonal (VWR GenScript A01622-40); western blot: 1:1,000.

### Nanoindentation

Mechanical properties at the microscopic scale were measured using nano-indentation (Piuma; Optics11, Amsterdam, The Netherlands) equipped with a dynamic mechanical analysis package (DMA). Freshly isolated cardiomyocytes were attached to glass-bottom dishes coated with MyoTak (lonOptix) in NT solution at 1 mM Ca2+. A spherical indentation probe with a radius of 4.5 µm and a stiffness of 0.1 N/m was used to indent the myocytes to a depth of 1-2 µm and the probe was then oscillated in a sinusoidal pattern at a frequency of 0.05, 0.1, 0.5, 1, 2, 5, 10, 20 and 50 Hz and an amplitude of 1 µm. The Piuma Data Viewer version 2.0 was used to convert DMA indentation depth and cantilever deflection into E' and E" and the magnitude of the elastic modulus was determined by E² = E'² + E"² A Poisson's ratio (n) of 0.5 was assumed and the average of E in each experimental condition was determined as a function of indentation speed.

Low-velocity indentation measures elastic contribution to stiffness, and high-velocity indentation measures elastic and viscous contributions. The change in modulus with rate represents myocyte viscoelasticity and can be fit to determine the model and magnitude of viscoelastic behavior.

### Isolated cardiomyocyte stretch and mechanical assessment

Either freshly isolated or cultured myocytes were diluted to a sparse density (~10x) into a large (22 x 50 mm) glass bottom petri dish coated with BSA to prevent myocyte adhesion with Normal Tyrodes solution. A small aliquot of Myotak^{™} was freshly thawed and a 1.5 uL droplet was placed in a dry area of the petri dish and allowed to polymerize (2-3 min). When the droplet had become "tacky" laser etched glass rods (Ion Optix LLC) were lowered into the solution several times to create a thin coating of Myotak which was allowed to dry for 90 seconds before the probes were submersed into the cell-containing bath. Myocytes were then attached to the glass holder by light contact and raised slightly above the coverslip before mechanical testing. The Myostretcher apparatus (Ion Optix LLC) was used to stretch myocytes by roughly 10% over either a 200 ms (diastolic) or 5 s (slow) interval while force and sarcomere length were recorded using the Ion Wizard software and MyoCam imaging system (live FFT). Each cell was subsequently imaged with transmitted light to obtain the cross-sectional area and convert forces into stresses. Following each protocol cells were removed by raising the cell holders out of solution several times, and an optical fiber was used to remove additional debris. If cells did not adhere well to the holders, probes were cleaned using a solution of Trypsin/EGTA, physically cleaned with an optical fiber, and recoated with Myotak.

### Immunofluorescence.

Cells were fixed in 4% PFA (Electron Microscopy Sciences) for 10 minutes, washed 3 times with PBS and permeabilized in 0.1% TritonX-100 for 10 minutes at room temperature. After washing twice with PBS, cells were placed in blocking buffer (1:1 Seablock (Abcam) and 0.1% TritonX-100 (Bio-Rad) in PBS) for at least 1 hour at room temperature, then labeled with primary antibodies (see below) for 24-48 hours at 4°C. Cells were then washed three times in TBS, then labeled with secondary antibodies (see below) in TBS at room temperature for 2-4 hours and washed a final two times with TBS. Stained cells were mounted on #1.5 coverslips in Prolong Diamond Antifade Mountant (Thermo Fisher) for imaging. Alternatively, cells were imaged directly in chambers with TBS. Imaging was carried out on a Zeiss 880 Airyscan confocal microscope operating on an Axiovert Z1 inverted microscope equipped with a Plan-Apochromat 63x oil 1.4 NA objective. Image analysis was performed using ZEN Black software for Airyscan processing, which involves signal integration from the 32 separate sub-resolution detectors in the Airyscan detector and subsequent deconvolution of this integrated signal.

### Transthoracic Echocardiography

Echocardiography measurements were acquired Vevo2100 Ultrasound System (VisualSonics Inc., Toronto, Ontario, Canada) with a MS250 (13-24 MHz) transducer. Animals were sedated with inhaled isoflurane (2-3%). Parasternal long axis, parasternal short axis, apical four chamber, and apical two chamber views were acquired to assess cardiac structure and function. Diastology was assessed using pulse wave and tissue doppler imaging. The protocol used was based on recommendations from the European Society of Cardiology Working Group on Myocardial Function (Zacchigna S *et al.* 2021).

### Invasive Hemodynamics

Animals were induced with isoflurane (5%) before being intubated and mechanically supported. Anesthesia plane was maintained using isoflurane (2-3%). A 2-French pressure-conductance catheter (Transonic Systems Inc., Ithaca, New York, USA) was calibrated using echo derived left ventricular volumes. The right common carotid artery was then dissected, and the catheter was advanced in a retrograde fashion into the left ventricle as previously described. Data were acquired using PowerLab and LabChart Pro (ADInstruments) and analyzed offline using LabChart Pro (ADInstruments).

### Results and discussion

### A validated target for heart failure treatment

The proteomic profiling of myocardial tissue collected from a cohort of patients with HCM who had undergone septal myectomy for symptomatic LVOTO revealed that many of the abnormalities in patients with HCM are similar among those with and without an identified sarcomere protein mutation (Schuldt M et al. Circ Heart Fail. 2021; 14:39-55). However, notable exceptions were abnormalities in cytoskeletal proteins and particularly the abundance of detyrosinated α-tubulin, which was much greater among patients with HCM, and a known sarcomere gene mutation than in those without an identified mutation. This particular finding is notable because recent studies have shown that this specific posttranslational modification-detyrosination of α-tubulin-has significant effects on the stability and density of the cardiomyocyte cytoskeleton and cell biomechanics. This specific increased detyrosination and associated changes to the cardiomyocyte microtubule network are causally linked to increased stiffness and viscoelasticity that reduce contractility and slow both contraction and relaxation. Microtubules are extensively altered as part of both adaptive and pathological cardiac remodeling, which has diverse ramifications for the structure and function of the cardiomyocyte.

### Distribution of tubulin detyrosination in myocardial tissue

We therefore sought to describe the distribution of tubulin detyrosination in myocardial tissue from healthy animals (Fig. 1). Using our affinity purified antibody that specifically detects detyrosinated alpha tubulin (Van der Laan et al., 2019), we optimised immunohistochemistry staining on formaldehyde embedded left ventricular cardiac tissue. While microtubules densification has been demonstrated (Cheng G. et al., 2008) as causative for hypertrophy and increased stiffness, healthy ventricular cardiomyocytes display a typical homogenous staining pattern with both longitudinal and transverse detyrosinated microtubules as previously shown on isolated cardiomyocytes (Robison et al. 2016). Of note, we could also see an increased staining level in the exterior layer of the heart tissue.

### Design and synthesis of Specific VASH Compounds (SVC)

Since to date no small molecule has been designed to inhibit the enzyme VASH that is responsible for tubulin detyrosination, we engineered various compounds based on the natural substrate of the enzyme. We undertook an extensive analysis of the human proteome and sought to identify human proteins that encoded a -EEY sequence at the very C-terminal. Only 3 proteins matched the criteria including alpha-tubulin. Therefore, we developed 5 compounds that all share a tyrosine in the structure that is essential for the specificity (Fig.2). We engineered prodrugs that are inactive on the therapeutic target in the context of in vitro detyrosination assays using recombinant protein human VASH1 (Fig 3) and its natural substrate. Using computer-aided drug discovery and iterative cycles of medicinal chemistry work-up followed by ELISA (in vitro) and HCS (in cellulo) testing, we gathered a SAR database containing new chemical entities. The potency and selectivity of these compound is considered within pharmaceutical standards for further development. Our initial prototype inhibitor (Epo-Y) shows dose-dependent inhibition of the detyrosinase activity. That said, the newly developed Specific VASH Compound (SVC) named SVC_01 displays about 100x better inhibition compared to Epo-Y. The prodrug SVC_02 is inactive toward the therapeutic target and presentation of SVC_02 to the reactive mixture does not induce reduction of the activity. Next, using a human cell-line we assayed the activity of the compound SVC_01 in a cellular system that further gives indication on penetration and cellular efficacy. Following 2 hours exposure of the human cells to the SVC_01 compound, we fixed and analysed by specific immunofluorescence the labelling of detyrosinated tubulin but also that of another posttranslational modification termed acetylation (Fig. 4). We compared the level of staining to a human double knock-out VASH cell-line that does not have detectable levels of detyrosinated tubulin. Our specific VASH compound SVC_01 completely reduced the level of tubulin detyrosination in human cells without affecting the level of acetylation, further demonstrating the specific approach. Although specific, SVC_ 01 was used at micromolar concentration to obtain full inhibition. With the objective to improve cellular IC50 of our SVCs, we designed compounds that have better cell penetration specifications. In turn, we obtained SVC_02 and SVC_03 that both act as prodrugs and are ineffective on recombinant VASH1 enzyme but highly efficient on human cells (low nanomolar IC50 (Fig. 5). While the substrate is rather unique in the human proteome (ending with -EEY), we undertook the specificity analysis by selecting different cysteine protease and tested their activity in presence of SVCs. We choose the proteases activity based on the different proteolytic mechanism.

Below, the criteria for selection are detailed:
- Protease with documented carboxypeptidase activity towards alpha tubulin. Carboxypeptidase A hydrolyzes peptide bonds of C-terminal residues with aromatic or aliphatic side-chains (Fig. 6).
- Cysteine protease with preference for non-aromatic residues at P1'. The optimum recognition site for Human rhinovirus (HRV) type 14 3C protease is LEVLFQ/GP, with a glycine at the P1' position (Fig. 7).
- Cysteine protease with preference for aromatic residues at P1' site. Alike VASH, cathepsin B prefers an aromatic residue at the P1' position. This is considered to be our best control for specificity (Fig. 8)

None of these proteases were inhibited in presence of high micromolar range presence of the SVCs, further supporting the specificity of these compounds towards the therapeutic target VASH1.

### SVCs have a superior safety profile compared to microtubule binding drugs.

Next, we performed safety pharmacology experiments and head-to-head compared the cellular toxicity of SVCs on human cells. For our comparison we used microtubule binding agents including Parthenolide that previously was coined as an inhibitor of detyrosination (Fonrose et al., 2007). This compound was latter shown to disrupt microtubules by non-specific binding to tubulin in the molecular structure (Hotta T. et al., 2021). Using a human cell-line we observed profound cell viability issues in the micromolar concentration range while using both Parthenolide and Taxol (Fig. 9). On the other hand, no toxicity observed for SVC_01 even at concentrations as high a 100micromolar and following multiple days of exposure. Mitochondrial health was assayed using the Seahorse equipment XF. Seahorse XF Analyzers measure oxygen consumption rate (OCR) and extracellular acidification rate (ECAR) of live cells in a multi-well plate, interrogating key cellular functions such as mitochondrial respiration and glycolysis. XF Analyzers perform compound addition and mixing, label-free analytical detection, and automatic measurement of OCR and ECAR in real time. No mitochondrial defects were measured at 20 micromolar concentration of our VASH inhibitor (Fig. 10). With anticipation of the cardiac application our active pharmaceutical ingredients (API), we also performed hERG analysis using our SVCs. The human ether-a-go-go related gene (hERG) encodes the inward rectifying voltage gated potassium channel in the heart (IKr) which is involved in cardiac repolarisation. Dose-response in presence of SVC_03 did not identify any potential cardiotoxicity (Fig 12).

### Proofs of concept and efficacy in preclinical model

For efficacy testing we presented SVC_01 to primary cardiomyocytes isolated from Sprague Dawley rats. As we previously found in human cells (Figs. 4&5), dose-dependent inhibition of tubulin detyrosination was observed with close to 80% reduction following 2 hours incubation with SVC_01 (Fig. 12). This finding further corroborates that VASH is the main detyrosinase in these cell-type and that our newly designed specific VASH compounds are highly effective in engaging the target. Next, we performed functional studies by measuring key cardiomyocytes parameters including resting, fractional shortening, contraction time and relaxation time. We observed dose-dependent and statistically significant improvements of contraction and relaxation times in presence of SVC_02 (Fig. 13). The ZSF1 rat model is a hybrid rat obtained by a cross between a ZDF female and an SHHF male rat. This model is an established HFpEF rat model. Obese ZSF1 (O-ZSF1) hybrid rats are compound heterozygote with two different mutated alleles coding defective leptin receptor, resulting in loss of the receptor function. Thus, these animals have increased food intake and develop pronounced features of obesity, diabetes, and resultant HFpEF. ZSF1 rats that have inherited two wild type alleles or only one of the parental mutated alleles, have a normal leptin signalling, balanced food intake and energy expenditure, normal weight, and do not develop disease traits (L-ZSF1). We performed a western blot analysis from myocardial tissue prepared from Wistar Kyoto (WKY), ZSF1 lean and ZSF1 obese rats (fig. 14). In line with previously published data of the role of microtubule densification and tubulin detyrosination, we found an increase in both total tubulin as well as detyrosinated tubulin in myocardial tissue of ZSF1-obese rats compared to the lean and WKY controls. Isolated cardiomyocytes from ZSF1 rats incubated with SVC_01 showed significant reduction of tubulin detyrosination (fig. 15). Moreover, treatment with our VASH inhibitor improved functional parameters of the isolated sick cardiomyocytes of 30-weeks aged obese ZSF1 rats. Both contraction and relaxation kinetics were improved following 2-hours treatment with the compound. The beneficial effect of the treatment was similar to that obtained with adenovirus transfected cardiomyocytes overexpressing TTL, the reverse enzyme responsible for re-tryosination, hence reducing detyrosination (Fig. 16). The functional improvement was statistically significant and of importance vehicle treated cardiomyocytes isolated from obese rats displayed significant functional impairment. Similar results were observed by assessment of the transverse stiffness. Indeed, VASHi treatment as TTL overexpression both led to marked and strong functional improvement of the cardiomyocyte transverse and longitudinal stiffness (Figs. 17,18). To test the efficacy of the treatment in reducing clinically relevant cardiac outcomes, we tail vain injected the compound (dose 6.6mg/kg) in Sprague dawley rats and collected cardiac tissue for western blot analysis. 8 hours following intravenous injection, a clear and significant reduction of tubulin detyrosination was observed in the myocardial tissue (Fig. 19). The same administration route was used for the ZSF1 rat model to engage the therapeutic target in the heart tissue. We found about 60% reduction of tubulin detyrosination in both lean and obese animals 8 hours following intravenous injection of SVC_02 (fig 20). The primary outcome is a significant reduction in Tau, mitral valve deceleration time and a trend towards significant reduction in LVDEP following acute IV injection of VASHi (Fig21).

In response to stress provocations, ZSF1 obese rats have poorer peak exercise tolerance and a steeper rate-dependent increase in filling pressures. SVCs reduced microtubule detyrosination in hearts from ZSF1 obese rats. This led to faster relaxation as judged by significant improvements of mitral valve deceleration time (A) and left ventricle relaxation time constant TAU (B), a trend towards lower spontaneous LVEDP, and a tempered provokable increase in LVEDP. Altogether, these results therefore validate the ZSF1 rat as a model for HFpEF, and importantly demonstrate that reducing microtubule detyrosination using SVCs has therapeutic potential in mitigating impaired relaxation in HFpEF and is a valid strategy for prevention or treatment of heart failure.

### REFERENCES

Aillaud, C. et al. Vasohibins/SVBP are tubulin carboxypeptidases (TCPs) that regulate neuron differentiation. Science 358, 1448-1453 (2017).
Caporizzo, M. A., Chen, C. Y. & Prosser, B. L. Cardiac microtubules in health and heart disease. Experimental Biology and Medicine 260, 1535370219868960 (2019).
Chen, C.Y et al. Suppression of detyrosinated microtubules improves cardiomyocyte function in human heart failure. Nature Medicine, volume 24, pages1225-1233 (2018), doi.org/10.1038/s41591-018-0046-2.
Chen, C. Y. et al. Depletion of Vasohibin 1 Speeds Contraction and Relaxation in Failing Human Cardiomyocytes. Circulation Research 116, 2662 (2020).
Cheng G. et al., A direct test of the hypothesis that increased microtubule network density contributes to contractile dysfunction of the hypertrophied heart. Am J Physiol Heart Circ Physiol. 2008 May; 294(5):H2231-41. doi: 10.1152/ajpheart.91515.2007.
Fonrose et al., (2007). "Parthenolide inhibits Tubulin Carboxypeptidase Activity", Cancer research, vol.67, n°7, 1 April 2007, pages 3371-3378.
Hotta T. et al., Parthenolide Destabilizes Microtubules by Covalently Modifying Tubulin. Curr Biol. 2021 Feb 22;31(4):900-907.e6. doi: 10.1016/j.cub.2020.11.055.
Kerr, J. P. et al. Detyrosinated microtubules modulate mechanotransduction in heart and skeletal muscle. Nat Commun 1-14 (2015). doi:10.1038/ncomms9526
McDonagh T.A et al., 2021 ESC Guidelines for the diagnosis and treatment of acute and chronic heart failure. Eur Heart J. 2021 Sep 21;42(36):3599-3726. doi: 10.1093/eurheartj/ehab368.
Maron, B.J et al. Prevalence of hypertrophic cardiomyopathy in a general population of young adults. Echocardiographic analysis of 4111 subjects in the CARDIA Study. Coronary Artery Risk Development in (Young) Adults. Circulation. 1995 Aug 15;92(4):785-9. doi: 10.1161/01.cir.92.4.785.
Robison, P. et al. Detyrosinated microtubules buckle and bear load in contracting cardiomyocytes. Science 352, aaf0659-aaf0659 (2016).
Roll-Mecak A., The Tubulin Code in Microtubule Dynamics and Information Encoding, Dev Cell, 2020 Jul 6;54(1):7-20. doi: 10.1016/j.devcel.2020.06.008.
Schuldt, M. & Kuster, D. W. D. Proteomic and functional studies reveal detyrosinated tubulin as treatment target in sarcomere mutation-induced hypertrophic cardiomyopathy. Circulation: Heart Failure (2020).
Tsutsui H. et al., Cytoskeletal role in the contractile dysfunction of hypertrophied myocardium. Science, 1993 Apr 30;260(5108):682-7. doi: 10.1126/science.8097594.
Van der Laan S. et al., Evolutionary Divergence of Enzymatic Mechanisms for Tubulin Detyrosination. Cell Reports. Volume 29, Issue 12, 17 December 2019, Pages 4159-4171.e6. doi.org/10.1016/j.celrep.2019.11.074.
Yu X. et al., MARK4 controls ischaemic heart failure through microtubule detyrosination. Nature, volume 594, pages560-565 (2021). doi :10.1038/s41586-021-03573-5
Zacchigna S, Paldino A, Falcao-Pires I, Daskalopoulos EP, Dal Ferro M, Vodret S, Lesizza P, Cannata A, Miranda-Silva D, Lourenco AP, et al. Towards standardization of echocardiography for the evaluation of left ventricular function in adult rodents: a position paper of the ESC Working Group on Myocardial Function. Cardiovasc Res. 2021;117:43-59. doi: 10.1093/cvr/cvaa110.

## Claims

1. A compound of formula (I) : 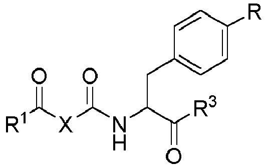
or a pharmaceutically acceptable salt and/or solvate thereof,
in which
X is 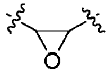 or -NH-CH₂-
R1 is 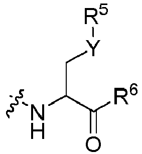 or NR^{1a}R^{1b} with R^{1a} is H and R^{1b} is C₁- C₆ alkyl non substituted or substituted with C(O)-O-C₁-C₆ alkyl or C(O)-NH-Ci-Ce alkyl,
R is O-R² with R² is a C₁-C₆ aliphatic chain, said aliphatic chain being optionally substituted, or C₁-C₆ alkyl-aryl,
R³ is OH or O-C₁-C₆ aliphatic chain,
Y is -(CH₂)ₘ- with m=2 or 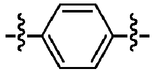
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain,
for use in the prevention and/or the treatment of heart failure, cardiomyopathies, myocardial infarction induced cardiac dysfunction and/or for improving heart function in a subject in need thereof.

2. The compound of formula (I) for use according to claim 1, wherein the compound of formula (I) is formulated in a pharmaceutical composition with a pharmaceutically acceptable excipient.

3. The compound of formula (I) for use according to claim 2, wherein the pharmaceutical composition is intended for oral or parenteral administration.

4. The compound of formula (I) for use according to anyone of claims 1 to 3, wherein in the compound of formula (I),
X is 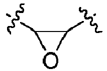
R1 is 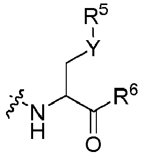 or NR^{1a}R^{1b} with R^{1a} is H and R^{1b} is C₁- C₆ alkyl non substituted or substituted with C(O)-O-C₁-C₆ alkyl or C(O)-NH-Ci-Ce alkyl,
R is O-R² with R² is a C₁-C₆ aliphatic chain, said aliphatic chain being non substituted or substituted with a phenyl, or C₁-C₆ alkyl-aryl,
R³ is OH or O-C₁-C₆ aliphatic chain,
Y is -(CH₂)ₘ- with m=2 or 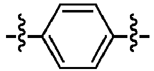
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain.

5. The compound of formula (I) for use according to anyone of claims 1 to 3, wherein in the compound of formula (I),
X= -NH-CH₂-
R¹ is NR^{1a}R^{1b} with R^{1a} is H and R^{1b} is C₁-Calkyl substituted with C(O)-NH-C₁-C₆ alkyl ,
R is O-R² with R² is C₁-C₆ alkyl-aryl, and
R³ is O-C₁-C₆ aliphatic chain.

6. The compound of formula (I) for use according to claim 4, wherein in the compound of formula (I),
R1 is 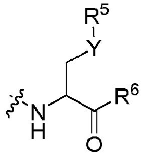 and
R is O-R² with R² is a C₁-C₆ aliphatic chain, said aliphatic chain being non substituted or substituted with a phenyl,
R³ is OH or O-C₁-C₆ aliphatic chain,
Y is -(CH₂)ₘ- with m=2 or 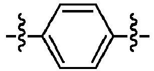
R⁵ is C(O)OH or O-C₁-C₆ alkyl, and
R⁶ is NH-CH(R⁷)-(CH₂)ₙ-R⁸ with R⁷ is H and R⁸ is aryl and n is 1, 2 or 3, preferably 1, or O-C₁-C₆ aliphatic chain.

7. The compound of formula (I) for use according anyone of claims 1 to 6, wherein the compound of formula (I) is selected from the following compounds: 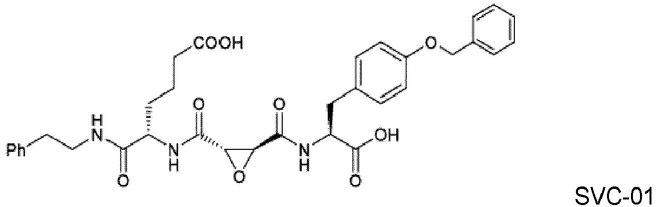 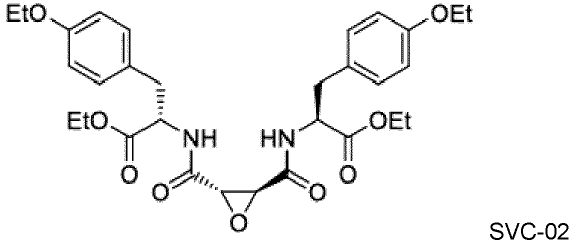 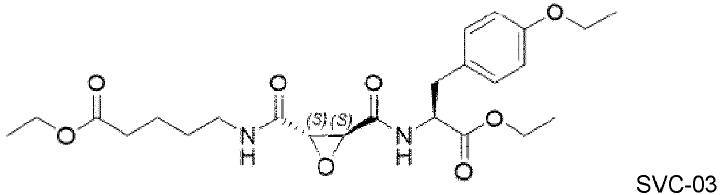 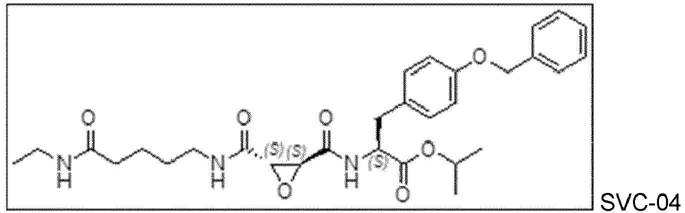 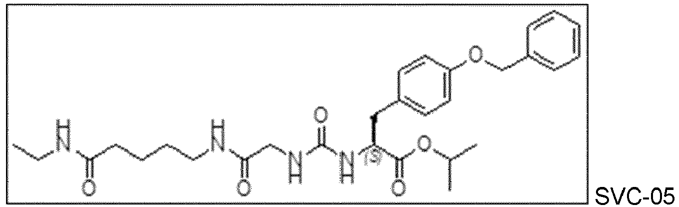 And 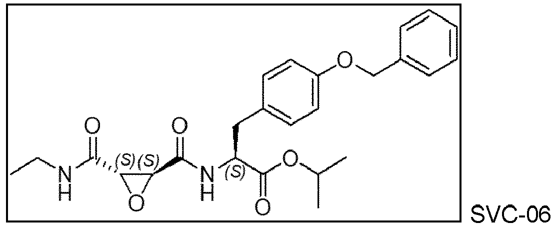 or conjugates thereof.

8. The compound of formula (I) for the use according to anyone of claims 1 to 7, wherein the compound of formula (I) improves kinetics of failing cardiomyocytes including increasing relaxation speed, increasing contractility, and/or reducing viscoelasticity.

9. The compound of formula (I) for the use according to anyone of claims 1 to 8, wherein the subject in need thereof has a heart failure with reduced ejection fraction (HFrEF).

10. The compound of formula (I) for the use according to anyone of claims 1 to 8, wherein the subject in need thereof has a heart failure with preserved ejection fraction (HFpEF).

11. The compound of formula (I) for the use according to anyone of claims 1 to 10, wherein the compound of formula (I) is administered to the subject in need thereof in combination with a current standard-of-care treatment for heart failure, cardiomyopathies, or myocardial infarction induced cardiac dysfunction.

12. A combination product comprising:
a) A compound for formula (I) as defined in anyone of claims 1, 4 to 7, or a pharmaceutical composition as defined in claims 2 or 3, and
b) A current standard-of-care treatment for heart failure, cardiomyopathies, or myocardial infarction induced cardiac dysfunction.

13. A use of the compound (I) as defined in claim 1 or a conjugate comprising a fragment of a compound of formula (I) linked or not to a biomolecule optionally labeled, as research tool for research and development activities in heart function disorders or heart failure.
